(19) Europäisches Patentamt
European Patent Office
Office européen des brevets

(11) EP 1 210 951 B1

(12) **EUROPEAN PATENT SPECIFICATION**

(45) Date of publication and mention of the grant of the patent:
**02.02.2005 Bulletin 2005/05**

(21) Application number: **01309457.8**

(22) Date of filing: **08.11.2001**

(51) Int Cl.[7]: **A61P 3/06**, A61P 5/26, A61P 5/30, A61P 5/32, A61P 9/10, A61P 13/08, A61P 19/10, A61P 43/00, A61P 15/12, A61K 31/444, A61K 31/4709, A61K 31/472, A61K 31/4725, A61K 31/4453, A61K 31/439, A61K 31/568, A61K 31/40, A61K 31/565, A61K 31/44
// (A61K31/565, 31:40)

(54) **Composition containing estrogen agonists/antagonists and testosterone for treating a decline in the level of the hormone testosterone**

Östrogen Agonist-Antagonist und Testosteron enthaltende Zusammensetzung zur Behandlung abnehmender Testosteronspiegel

Composition contenant des agoniste-antagonistes d'oestrogène et de la testostérone pour le traitement d' une réduction des taux de l'hormone testostérone

(84) Designated Contracting States:
**AT BE CH CY DE DK ES FI FR GB GR IE IT LI LU MC NL PT SE TR**

(30) Priority: **30.11.2000 US 250071 P**

(43) Date of publication of application:
**05.06.2002 Bulletin 2002/23**

(73) Proprietor: **Pfizer Products Inc.**
**Groton, Connecticut 06340 (US)**

(72) Inventor: **McLean, David Burton,**
**c/o Pfizer Global Res.& Dev.**
**Groton, Connecticut 06340 (US)**

(74) Representative: **Hayles, James Richard et al**
**Pfizer Limited,**
**Patents Department,**
**Ramsgate Road**
**Sandwich Kent CT13 9NJ (GB)**

(56) References cited:

| | |
|---|---|
| **EP-A- 0 793 961** | **EP-A- 0 888 775** |
| **WO-A-97/31640** | **DE-A- 19 825 591** |
| **US-A- 5 552 412** | **US-A- 5 855 905** |

- **WEISSBERGER ANDREW J ET AL: "Activation of the somatotropic axis by testosterone in adult males: Evidence for the role of aromatization." JOURNAL OF CLINICAL ENDOCRINOLOGY & METABOLISM, vol. 76, no. 6, 1993, pages 1407-1412, XP002247731 ISSN: 0021-972X**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

### Field of the Invention

The present invention concerns the use of a combination of an estrogen agonist / antagonist and testosterone for the manufacture of a medicament for treating a decline in the level of the hormone testosterone and related conditions, and provides kits for treating a decline in the level of the hormone testosterone and the conditions related to or resulting from a decline in the level of the hormone testosterone.

### Background of the Invention

Andropause (also called male menopause or viropause) is a natural occurrence in men that typically happens between the age of forty and fifty-five. Andropause is a decline in the level of the hormone testosterone. As testosterone levels decline, and men enter andropause, various changes or conditions may be observed including decreased energy and strength, increased body fat, osteoporosis, depression, decreased mental acuity, inability to maintain muscle, cardiovascular disease, atherosclerosis, decreased libido, decreased strength of orgasms, erectile dysfunction, increased irritability, and aching and stiff joints, particularly in the hands and feet. In addition, males undergoing or having undergone andropause can have gynecomastia, serum lipid disorders, including hypercholesterolemia, reduced vascular reactivity, hypogonadism, and benign prostatic hyperplasia.

The present invention provides the use of a combination of an estrogen agonist/antagonist compound and testosterone for the manufacture of a medicament for treating a decline in the level of the hormone testosterone and the conditions associated with a decline in the level of the hormone testosterone, and also provides kits for treating a decline in the level of the hormone testosterone and the conditions associated with a decline in the level of the hormone testosterone.

Journal of Clinical Endocrinology and Metabolism, 1993., 76, 6., 1407-1412, discusses the role of testosterone in the modulation of the male somatotropic axis in adulthood. EP0793961 discloses the use of droloxifene for the manufacture of a medicament for increasing serum levels of testosterone. US5,855,905 describes a compound preparation for the treatment of hypogonadal men and men with hypophyseal disease. EP0888775 discloses the control of selective estrogen receptor modulators. DE19825591 describes a pharmaceutical combination for use in treating testosterone deficient males. W097/31640 discloses a combination therapy for treating osteoporosis.

### Summary of the Invention

The present invention provides the use of a therapeutically effective amount of combination of an estrogen agonist/antagonist compound and testosterone for the manufacture of a medicament for treating a decline in the level of the hormone testosterone, gynecomastia, lipid disorders, cardiovascular disease, atherosclerosis, hypogonadism, benign prostatic hyperplasia, osteoporosis, decreased libido, or decreased vascular reactivity in a male patient.

The estrogen agonist / antagonist is a compound of formula I

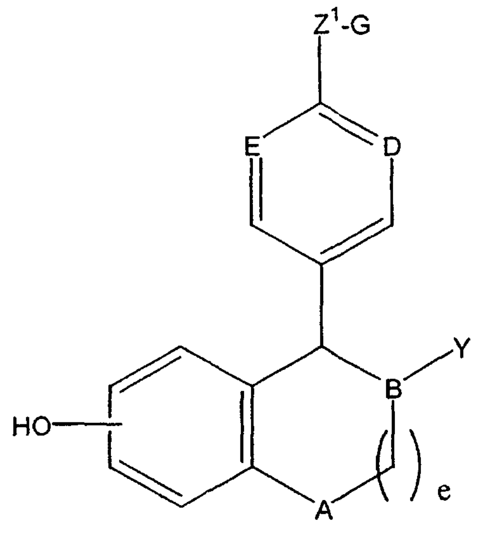

(I)

wherein:

A is selected from $CH_2$ and NR;
B, D and E are independently selected from CH and N;
Y is

(a) phenyl, optionally substituted with 1-3 substituents independently selected from $R^4$;
(b) naphthyl, optionally substituted with 1-3 substituents independently selected from $R^4$;
(c) $C_3$-$C_8$ cycloalkyl, optionally substituted with 1-2 substituents independently selected from $R^4$;
(d) $C_3$-$C_8$ cycloalkenyl, optionally substituted with 1-2 substituents independently selected from $R^4$;
(e) a five membered heterocycle containing up to two heteroatoms selected from the group consisting of -O-, $-NR^2-$ and $-S(O)_n-$, optionally substituted with 1-3 substituents independently selected from $R^4$;
(f) a six membered heterocycle containing up to two heteroatoms selected from the group consisting of -O-, $-NR^2-$ and $-S(O)_n-$ optionally substituted with 1-3 substituents independently selected from $R^4$; or
(g) a bicyclic ring system consisting of a five or six membered heterocyclic ring fused to a phenyl ring, said heterocyclic ring containing up to two heteroatoms selected from the group consisting of -O-, $-NR^2-$ and $-S(O)_n-$, optionally substituted with 1-3 substituents independently selected from $R^4$;

$Z^1$ is

(a) $-(CH_2)_p W(CH_2)_q-$;
(b) $-O(CH_2)_p CR^5R^6-$;
(c) $-O(CH_2)_pW(CH_2)_q-$;
(d) $-OCHR^2CHR^3-$; or
(e) $-SCHR^2CHR^3-$;

G is

(a) $-NR^7R^8$;
(b)

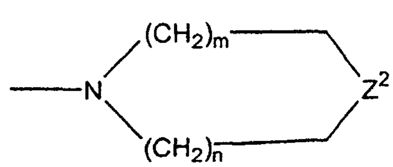

wherein n is 0, 1 or 2; m is 1, 2 or 3; $Z^2$ is -NH-, -O-, -S-, or $-CH_2-$; optionally fused on adjacent carbon atoms with one or two phenyl rings and, optionally independently substituted on carbon with one to three substituents and, optionally, independently on nitrogen with a chemically suitable substituent selected from $R^4$; or
(c) a bicyclic amine containing five to twelve carbon atoms, either bridged or fused and optionally substituted with 1-3 substituents independently selected from $R^4$; or

$Z^1$ and G in combination may be

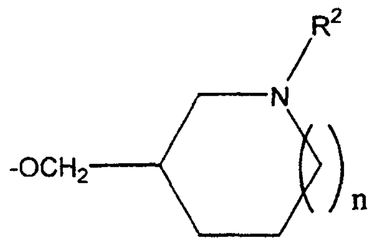
;

Wis

(a) $-CH_2-$;
(b) -CH=CH-;
(c) -O-;
(d) $-NR^2-$;
(e) $-S(O)_n-$;
(f)

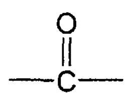
;

(g) $-CR^2(OH)-$;
(h) $-CONR^2-$;
(i) $-NR^2CO-$;
(j)

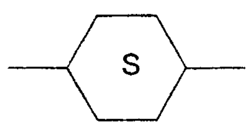
;

or
(k) -C≡C-;

R is hydrogen or $C_1$-$C_6$ alkyl;
$R^2$ and $R^3$ are independently

(a) hydrogen; or
(b) $C_1$-$C_4$ alkyl;

$R^4$ is

(a) hydrogen;
(b) halogen;
(c) $C_1$-$C_6$ alkyl;
(d) $C_1$-$C_4$ alkoxy;

(e) $C_1$-$C_4$ acyloxy;
(f) $C_1$-$C_4$ alkylthio;
(g) $C_1$-$C_4$ alkylsulfinyl;
(h) $C_1$-$C_4$ alkylsulfonyl;
(i) hydroxy ($C_1$-$C_4$)alkyl;
(j) aryl ($C_1$-$C_4$)alkyl;
(k) $-CO_2H$;
(l) -CN;
(m) -CONHOR;
(n) $-SO_2NHR$;
(o) $-NH_2$;
(p) $C_1$-$C_4$ alkylamino;
(q) $C_1$-$C_4$ dialkylamino;
(r) $-NHSO_2R$;
(s) $-NO_2$;
(t) -aryl; or
(u) -OH;

$R^5$ and $R^6$ are independently $C_1$-$C_8$ alkyl or together form a $C_3$-$C_{10}$ carbocyclic ring;
$R^7$ and $R^8$ are independently

(a) phenyl;
(b) a $C_3$-$C_{10}$ carbocyclic ring, saturated or unsaturated;
(c) a $C_3$-$C_{10}$ heterocyclic ring containing up to two heteroatoms, selected from -O-, -N- and -S-;
(d) H;
(e) $C_1$-$C_6$ alkyl; or
(f) form a 3 to 8 membered nitrogen containing ring with $R^5$ or $R^6$;

$R^7$ and $R^8$ in either linear or ring form may optionally be substituted with up to three substituents independently selected from $C_1$-$C_6$ alkyl, halogen, alkoxy, hydroxy and carboxy;
a ring formed by $R^7$ and $R^8$ may be optionally fused to a phenyl ring;
e is 0, 1 or 2;
m is 1, 2 or 3;
n is 0, 1 or 2;
p is 0, 1, 2 or 3;
q is 0, 1, 2 or 3;

or an optical or geometric isomer thereof; or a pharmaceutically acceptable salt, N-oxide, ester, quaternary ammonium salt or prodrug thereof.

In another preferred embodiment of the methods, the estrogen agonist / antagonist is a compound of formula (IA)

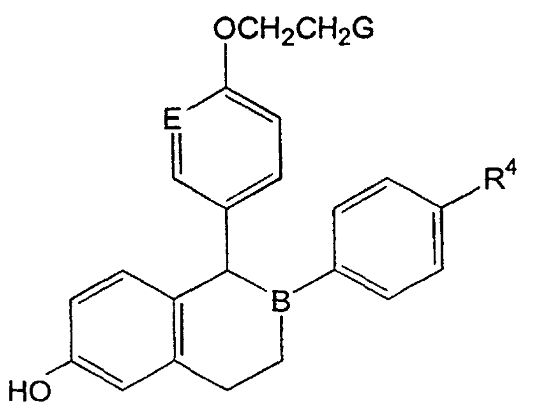

(IA)

wherein G is

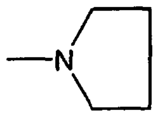 , 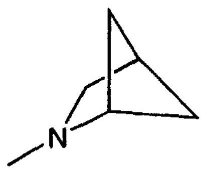 or 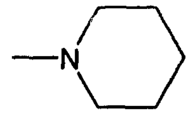 ;

$R^4$ is H, OH, F, or Cl; and B and E are independently selected from CH and N or an optical or geometric isomer thereof; or a pharmaceutically acceptable salt, N-oxide, ester, quaternary ammonium salt, or a prodrug thereof.

In a more preferred embodiment, the estrogen agonist / antagonist is (-)-cis-6-phenyl-5-[4-(2-pyrrolidin-1-yl-ethoxy)-phenyl]-5,6,7,8-tetrahydro-naphthalene-2-ol or an optical or geometric isomer thereof; a pharmaceutically acceptable salt, N-oxide, ester, quaternary ammonium salt, or a prodrug thereof.

In a still more preferred embodiment, the estrogen agonist / antagonist (-)-cis-6-phenyl-5-[4-(2-pyrrolidin-1-yl-ethoxy)-phenyl]-5,6, 7 ,8-tetrahydronaphthalene-2-ol is in the form of a D-tartrate salt.

In a preferred embodiment of the kits, the estrogen agonist / antagonist is a compound of formula I

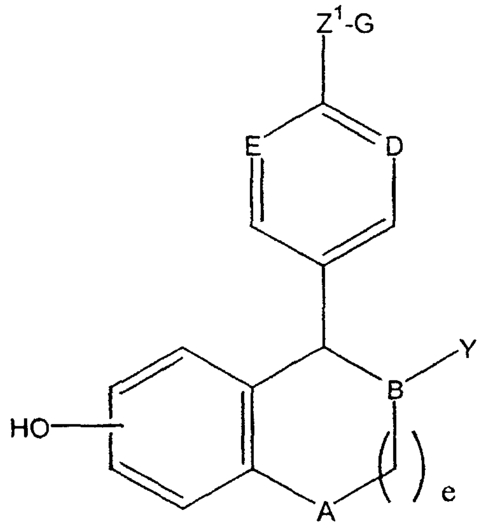

(I)

wherein:

A is selected from $CH_2$ and NR;
B, D and E are independently selected from CH and N;
Y is

(a) phenyl, optionally substituted with 1-3 substituents independently selected from $R^4$;
(b) naphthyl, optionally substituted with 1-3 substituents independently selected from $R^4$;
(c) $C_3$-$C_8$ cycloalkyl, optionally substituted with 1-2 substituents independently selected from $R^4$;
(d) $C_3$-$C_8$ cycloalkenyl, optionally substituted with 1-2 substituents independently selected from $R^4$;
(e) a five membered heterocycle containing up to two heteroatoms selected from the group consisting of -O-, $-NR^2-$ and $-S(O)_n-$, optionally substituted with 1-3 substituents independently selected from $R^4$;
(f) a six membered heterocycle containing up to two heteroatoms selected from the group consisting of -O-, $-NR^2-$ and $-S(O)_n-$ optionally substituted with 1-3 substituents independently selected from $R^4$; or
(g) a bicyclic ring system consisting of a five or six membered heterocyclic ring fused to a phenyl ring, said heterocyclic ring containing up to two heteroatoms selected from the group consisting of -O-, $-NR^2-$ and $-S(O)_n-$, optionally substituted with 1-3 substituents independently selected from $R^4$;

$Z^1$ is

(a) $-(CH_2)_p W(CH_2)_q-$;
(b) $-O(CH_2)_p CR^5R^6-$;
(c) $-O(CH_2)_pW(CH_2)_q-$;
(d) $-OCHR^2CHR^3-$; or
(e) $-SCHR^2CHR^3-$;

G is

(a) $-NR^7R^8$:
(b)

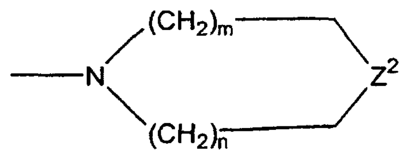

wherein n is 0, 1 or 2; m is 1, 2 or 3; $Z^2$ is -NH-, -O-, -S-, or $-CH_2-$; optionally fused on adjacent carbon atoms with one or two phenyl rings and, optionally independently substituted on carbon with one to three substituents and, optionally, independently on nitrogen with a chemically suitable substituent selected from $R^4$; or

(c) a bicyclic amine containing five to twelve carbon atoms, either bridged or fused and optionally substituted with 1-3 substituents independently selected from $R^4$; or

$Z^1$ and G in combination may be

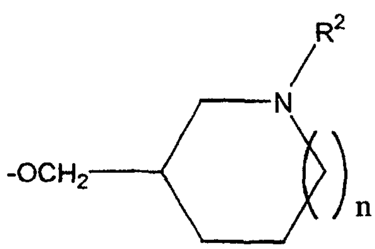

;

Wis

(a) $-CH_2-$;
(b) -CH=CH-;
(c) -O-;
(d) $-NR^2-$;
(e) $-S(O)_n-$;
(f)

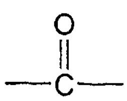

;

(g) $-CR^2(OH)-$;
(h) $-CONR^2-$;
(i) $-NR^2CO-$;
(j)

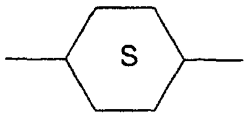
;

or
(k) -C≡C-;

R is hydrogen or $C_1$-$C_6$ alkyl;
$R^2$ and $R^3$ are independently

(a) hydrogen; or
(b) $C_1$-$C_4$ alkyl;

$R^4$ is

(a) hydrogen;
(b) halogen;
(c) $C_1$-$C_6$ alkyl;
(d) $C_1$-$C_4$ alkoxy;
(e) $C_1$-$C_4$ acyloxy;
(f) $C_1$-$C_4$ alkylthio;
(g) $C_1$-$C_4$ alkylsulfinyl;
(h) $C_1$-$C_4$ alkylsulfonyl;
(i) hydroxy ($C_1$-$C_4$)alkyl;
(j) aryl ($C_1$-$C_4$)alkyl;
(k) $-CO_2H$;
(l) -CN;
(m) -CONHOR;
(n) $-SO_2NHR$;
(o) $-NH_2$;
(p) $C_1$-$C_4$ alkylamino;
(q) $C_1$-$C_4$ dialkylamino;
(r) $-NHSO_2R$;
(s) $-NO_2$;
(t) -aryl; or
(u) -OH;

$R^5$ and $R^6$ are independently $C_1$-$C_8$ alkyl or together form a $C_3$-$C_{10}$ carbocyclic ring;
$R^7$ and $R^8$ are independently

(a) phenyl;
(b) a $C_3$-$C_{10}$ carbocyclic ring, saturated or unsaturated;
(c) a $C_3$-$C_{10}$ heterocyclic ring containing up to two heteroatoms, selected from -O-, -N- and -S-;
(d) H;
(e) $C_1$-$C_6$ alkyl; or
(f) form a 3 to 8 membered nitrogen containing ring with $R^5$ or $R^6$;

$R^7$ and $R^8$ in either linear or ring form may optionally be substituted with up to three substituents independently selected from $C_1$-$C_6$ alkyl, halogen, alkoxy, hydroxy and carboxy;
a ring formed by $R^7$ and $R^8$ may be optionally fused to a phenyl ring;
e is 0, 1 or 2;
m is 1, 2 or 3;
n is 0, 1 or 2;
p is 0, 1, 2 or 3;
q is 0, 1, 2 or 3;

or an optical or geometric isomer thereof; or a pharmaceutically acceptable salt, N-oxide, ester, quaternary ammonium salt or prodrug thereof.

In another preferred embodiment of the kits, the estrogen agonist / antagonist is a compound of formula (IA)

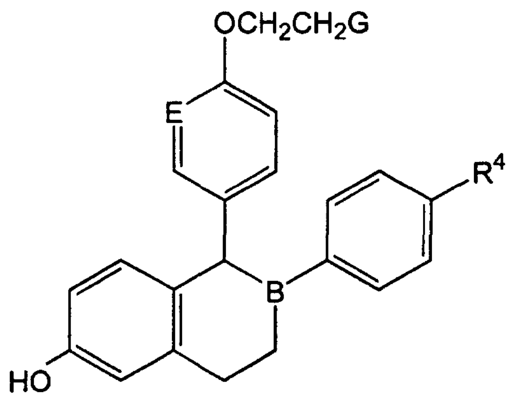

(IA)

wherein G is

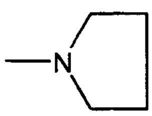 , 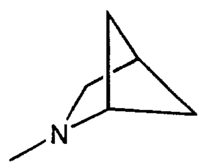 or 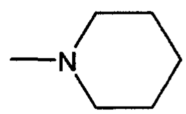 ;

$R^4$ is H, OH, F, or Cl; and B and E are independently selected from CH and N or an optical or geometric isomer thereof; or a pharmaceutically acceptable salt, N-oxide, ester, quaternary ammonium salt, or a prodrug thereof.

In a more preferred embodiment, the estrogen agonist / antagonist is (-)-cis-6-phenyl-5-[4-(2-pyrrolidin-1-yl-ethoxy)-phenyl]-5,6,7,8-tetrahydro-naphthalene-2-ol or an optical or geometric isomer thereof; a pharmaceutically acceptable salt, N-oxide, ester, quaternary ammonium salt, or a prodrug thereof.

In a more preferred embodiment, the estrogen agonist / antagonist is in the form of a D-tartrate salt.

In another preferred embodiment of the kits, the kits include one or more additional compounds that are useful for treating a decline in the level of the hormone testosterone, gynecomastia, lipid disorders, cardiovascular disease, atherosclerosis, hypogonadism, benign prostatic hyperplasia, or osteoporosis, or increasing libido, or maintaining or improving vascular reactivity in a male patient.

Detailed Description of the Invention

The present invention relates to the use of the compounds described above in combination with testosterone for the manufacture of a medicament for treating a decline in the level of the hormone testosterone and conditions that relate to a decline in the level of the hormone testosterone. The invention also relates to kits for treating a decline in the level of the hormone testosterone and conditions that relate to a decline in the level of the hormone testosterone.

The term "andropause" is known to those skilled in the art and relates to a period in an adult male's life when testosterone levels decrease significantly. Typically, testosterone levels decrease naturally as males age. Males who are past puberty usually have plasma testosterone concentrations in the range of about 300 to about 1200 ng/dL. During and after andropause, testosterone levels can drop to the range of about 200 to about 300 ng/dL or lower. Andropause is sometimes viewed as "male menopause." Medical conditions or accidents can also cause decreased testosterone levels, which can result in the conditions typically seen in connection with andropause. The treatments disclosed herein will also be applicable to these conditions. Andropause is determined by the existence of the conditions associated therewith and/or by decreased levels of testosterone.

The term "lipid disorders" includes hypercholesterolemia, high plasma concentrations of low density lipoprotein (LDL), and low plasma concentrations of HDL. Those skilled in the art are familiar with the lipid levels that can

result in pathological conditions (lipid disorders). For example, high plasma concentrations of LDL have been associated with an increased risk of developing cardiovascular disease, particularly atherosclerosis. The administration of an estrogen agonist / antagonist and testosterone can help maintain desired lipid levels and can help improve unacceptable lipid levels. During and after andropause, lipid disorders are seen with increased frequency.
Vascular reactivity relates to a blood vessel's ability to dilate and contract after being presented with certain stimuli. The ability of a blood vessel to react appropriately to stimuli is important. For example, constriction of blood vessels during an ischemic event results in further ischemia and can exacerbate the damage caused by the ischemia. During and after andropause, it is typical for vascular reactivity to decrease. The administration of an estrogen agonist / antagonist and testosterone can help prevent declines in vascular reactivity and can help improve diminished vascular reactivity.

The present invention also relates to increasing libido in a male. It is common for a male to have a decreased desire for sexual intercourse during and after andropause. This decreased desire for sexual intercourse (decreased libido) can be treated by administering an estrogen agonist / antagonist and testosterone. The administration of an estrogen agonist / antagonist and testosterone increases the desire for sexual intercourse.

The present invention also relates to treating gynecomastia using an estrogen agonist /antagonist and testosterone. Gynecomastia relates to the unwanted enlargement of the male breast. During and after andropause, a male may have unwanted enlargement of one or both of the breasts. This unwanted enlargement can be treated by administering an estrogen agonist /antagonist and testosterone.

The present invention also relates to treating hypogonadism. Hypogonadism relates to decreased functionality of the testes, which can result in reproductive insufficiency. In addition to occurring during or after andropause, hypogonadism can occur in a young male and can retard puberty. Various types of hypogonadism and conditions in which hypogonadism is seen have been identified including Klinefelter syndrome, bilateral anorchia, Leydig cells aplasia, Noonan syndrome, myotonic dystrophy, panhypopituitarism, Kallmann syndrome, and Prader-Willi syndrome. Hypogonadism can be treated by administering an estrogen agonist / antagonist and testosterone.

The present invention also relates to the treatment of benign prostatic hyperplasia (BPH). BPH, which causes bladder outlet obstruction, is the benign adenomatous hyperplasia of the periurethral prostate gland. The occurrence of BPH is increased during and after andropause. BPH can be treated by administering to a patient suffering therefrom an estrogen agonist / antagonist and testosterone.

The present invention also relates to the treatment of osteoporosis. Osteoporosis is a condition in which bone density decreases. The decrease in bone density results in skeletal weakness and increased frequency of fractures. Bone density is determined by the rate of bone formation and bone resorption. In osteoporosis, the rate of bone resorption exceeds the rate of bone formation. Thus, the treatment of osteoporosis relates to decreasing bone resorption, increasing bone formation or both. During or after andropause, the incidence of osteoporosis is increased. Osteoporosis can be treated by administering to a patient suffering therefrom an estrogen agonist / antagonist and testosterone.

The present invention also relates treating atherosclerosis. Atherosclerosis is the patchy, subintimal thickening of medium and large arteries, which can reduce or completely obstruct blood flow. Commonly, the patches are known as plaques. The size of a plaque is important. The larger the plaque, the more obstructed the blood flow. In addition to obstructed blood flow, a plaque can rupture, which can result in blood clots being formed that can reduce or completely obstruct blood flow. Reduced or obstructed blood flow can result in stroke or myocardial infarction. The incidence of atherosclerosis in males increases during and after andropause. Atherosclerosis can be treated by administering to a patient in need thereof an estrogen agonist / antagonist and testosterone. The administration of an estrogen agonist/ antagonist and testosterone helps prevent the formation of atherosclerotic plaques, helps prevent further growth of atherosclerotic plaques, and can help reduce the incidence of atherosclerotic plaque rupture.

The present invention also relates to the treatment of cardiovascular disease, including atherosclerosis. Cardiovascular disease relates primarily to conditions affecting the heart, veins and arteries. One aspect of cardiovascular disease concerns reduced blood flow to various organs. Reduced blood flow can be the result of many different causes. For example, an artery carrying blood to an organ can be blocked by an atherosclerotic plaque or a blood clot. The particular damage that occurs as a result of reduced blood flow depends on which organ is experiencing the reduced blood flow, which is also known as ischemia. If the blood flow is so reduced as to result in the death of a portion of the tissue of the heart, the term myocardial infarction (heart attack) is used. Likewise, reduced blood flow to the brain can result in a stroke. Other types of cardiovascular disease include coronary artery disease, which relates to atherosclerosis of the arteries supplying blood to the heart, and peripheral vascular disorders, including peripheral arterial occlusion, which is the obstruction of the blood supply to the extremities such as the legs, arms, hands and feet. The incidence of cardiovascular disease is increased during and after andropause. Cardiovascular disease can be treated by administering to a patient suffering therefrom an estrogen agonist /antagonist and testosterone.

The level of testosterone in a male can be measured by measuring the amount of testosterone in plasma. Since testosterone binds to certain plasma proteins, testosterone levels can be adjusted to take protein bound testosterone into account. Testosterone that is not bound to proteins is sometimes called free testosterone. Those skilled

in the art are familiar with the measurement of plasma testosterone levels, including plasma bound and free testosterone.

The terms "treat", "treatment", and "treating" include preventative (e.g., prophylactic) and palliative treatment or the act of providing preventative or palliative treatment.

The term "patient" means male animals, particularly mammals. Preferred patients are male humans over the age of fifty (i.e., elderly males).

An "estrogen agonist / antagonist" is a compound that affects some of the same receptors that estrogen does, but not all, and in some instances, it antagonizes or blocks estrogen. It is also known as a "selective estrogen receptor modulator" (SERM). Estrogen agonists / antagonists may also be referred to as antiestrogens although they have some estrogenic activity at some estrogen receptors. Estrogen agonists / antagonists are therefore not what are commonly referred to as "pure antiestrogens". Antiestrogens that can also act as agonists are referred to as Type I antiestrogens. Type I antiestrogens activate the estrogen receptor to bind tightly in the nucleus for a prolonged time but with impaired receptor replenishment (Clark, et al., Steroids 1973;22:707, Capony et al., Mol Cell Endocrinol, 1975;3:233).

The estrogen agonists / antagonists and testosterone of this invention may be administered systemically or locally. For systemic use, the estrogen agonists / antagonists herein are formulated for parenteral (e.g., intravenous, subcutaneous, intramuscular, intraperitoneal, intranasal or transdermal) or enteral (e.g., oral or rectal) delivery according to conventional methods. Intravenous administration can be by a series of injections or by continuous infusion over an extended period. Administration by injection or other routes of discretely spaced administration can be performed at intervals ranging from weekly to once to three or more times daily.

Another method of administering the compounds of the present combination includes the use of topical dosage forms. For example, the active agent or agents can be administered to a patient in a cream, ointment or gel that is applied to the skin. Alternatively, the active agents can be delivered using a patch that is applied to the skin.

Preferred estrogen agonists / antagonists of the present invention include the compounds described in US 5,552,412. Those compounds are described by the formula designated herein as formula (I) given below:

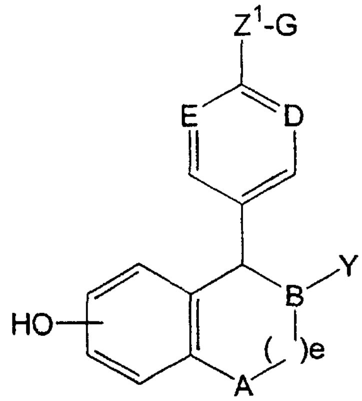

(I)

wherein:

A is selected from $CH_2$ and NR;
B, D and E are independently selected from CH and N;
Y is

(a) phenyl, optionally substituted with 1-3 substituents independently selected from $R^4$;
(b) naphthyl, optionally substituted with 1-3 substituents independently selected from $R^4$;
(c) $C_3$-$C_8$ cycloalkyl, optionally substituted with 1-2 substituents independently selected from $R^4$;
(d) $C_3$-$C_8$ cycloalkenyl, optionally substituted with 1-2 substituents independently selected from $R^4$;
(e) a five membered heterocycle containing up to two heteroatoms selected from the group consisting of -O-, -$NR^2$- and -$S(O)_n$-, optionally substituted with 1-3 substituents independently selected from $R^4$;
(f) a six membered heterocycle containing up to two heteroatoms selected from the group consisting of -O-, -$NR^2$- and -$S(O)_n$- optionally substituted with 1-3 substituents independently selected from $R^4$; or
(g) a bicyclic ring system consisting of a five or six membered heterocyclic ring fused to a phenyl ring, said heterocyclic ring containing up to two heteroatoms selected from the group consisting of -O-, -$NR^2$- and -$S(O)_n$-, optionally substituted with 1-3 substituents independently selected from $R^4$;

$Z^1$ is

(a) $-(CH_2)_p W(CH_2)_q-$;
(b) $-O(CH_2)_p CR^5R^6-$;
(c) $-O(CH_2)_pW(CH_2)_q-$;
(d) $-OCHR^2CHR^3-$; or
(e) $-SCHR^2CHR^3-$;

G is

(a) $-NR^7R^8$;
(b)

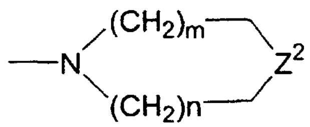

wherein n is 0, 1 or 2; m is 1, 2 or 3; $Z^2$ is -NH-, -O-, -S-, or $-CH_2-$; optionally fused on adjacent carbon atoms with one or two phenyl rings and, optionally independently substituted on carbon with one to three substituents and, optionally, independently on nitrogen with a chemically suitable substituent selected from $R^4$; or
(c) a bicyclic amine containing five to twelve carbon atoms, either bridged or fused and optionally substituted with 1-3 substituents independently selected from $R^4$; or

$Z^1$ and G in combination may be

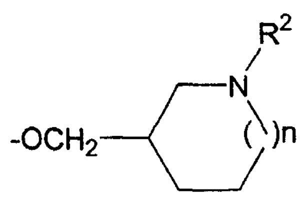
;

W is

(a) $-CH_2-$;
(b) -CH=CH-;
(c) -O-;
(d) $-NR^2-$;
(e) $-S(O)_n-$;
(f)

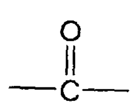
;

(g) $-CR^2(OH)-$;
(h) $-CONR^2-$;
(i) $-NR^2CO-$;
(j)

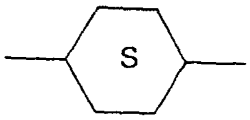
;

or
(k) -C≡C-;

R is hydrogen or $C_1$-$C_6$ alkyl;
$R^2$ and $R^3$ are independently

(a) hydrogen; or
(b) $C_1$-$C_4$ alkyl;

$R^4$ is

(a) hydrogen;
(b) halogen;
(c) $C_1$-$C_6$ alkyl;
(d) $C_1$-$C_4$ alkoxy;
(e) $C_1$-$C_4$ acyloxy;
(f) $C_1$-$C_4$ alkylthio;
(g) $C_1$-$C_4$ alkylsulfinyl;
(h) $C_1$-$C_4$ alkylsulfonyl;
(i) hydroxy ($C_1$-$C_4$)alkyl;
(j) aryl ($C_1$-$C_4$)alkyl;
(k) $-CO_2H$;
(l) -CN;
(m) -CONHOR;
(n) $-SO_2NHR$ ;
(o) $-NH_2$;
(p) $C_1$-$C_4$ alkylamino;
(q) $C_1$-$C_4$ dialkylamino;
(r) $-NHSO_2R$;
(s) $-NO_2$;
(t) -aryl; or
(u) -OH;

$R^5$ and $R^6$ are independently $C_1$-$C_8$ alkyl or together form a $C_3$-$C_{10}$ carbocyclic ring;
$R^7$ and $R^8$ are independently

(a) phenyl;
(b) a $C_3$-$C_{10}$ carbocyclic ring, saturated or unsaturated;
(c) a $C_3$-$C_{10}$ heterocyclic ring containing up to two heteroatoms, selected from -O-, -N- and -S-;
(d) H;
(e) $C_1$-$C_6$ alkyl; or
(f) form a 3 to 8 membered nitrogen containing ring with $R^5$ or $R^6$;

$R^7$ and $R^8$ in either linear or ring form may optionally be substituted with up to three substituents independently selected from $C_1$-$C_6$ alkyl, halogen, alkoxy, hydroxy and carboxy;
a ring formed by $R^7$ and $R^8$ may be optionally fused to a phenyl ring;
e is 0, 1 or 2;
m is 1, 2 or 3;
n is 0, 1 or 2;
p is 0, 1, 2 or 3;
q is 0, 1, 2 or 3;

and optical and geometric isomers thereof; and nontoxic pharmacologically acceptable acid addition salts, N-oxides, esters, quaternary ammonium salts and prodrugs thereof.

Additional preferred compounds are disclosed in U.S. Patent No. 5,552,412 and are described by the formula designated herein as formula (IA):

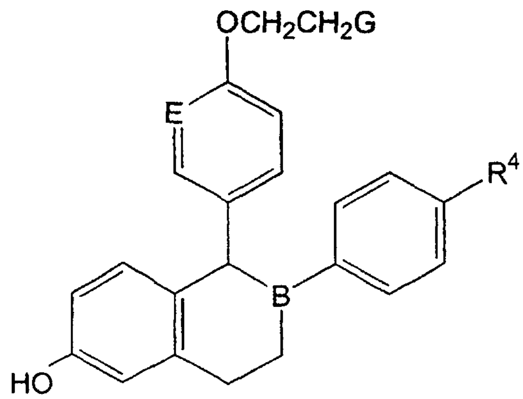

(IA)

wherein G is

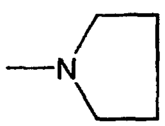

, 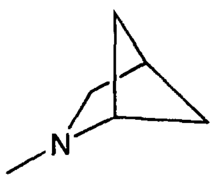

or 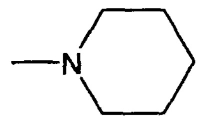

;

$R^4$ is H, OH, F, or Cl; and B and E are independently selected from CH and N, and optical and geometric isomers thereof; and nontoxic pharmacologically acceptable acid addition salts, N-oxides, esters, quaternary ammonium salts and prodrugs thereof.

Especially preferred estrogen agonists /antagonists for the methods and kits of the invention are:

cis-6-(4-fluoro-phenyl)-5-[4-(2-piperidin-1-yl-ethoxy)-phenyl]-5,6,7,8-tetrahydro-naphthalene-2-ol;
(-)-cis-6-phenyl-5-(4-(2-pyrrolidin-1-yl-ethoxy)-phenyl]-5,6,7,8-tetrahydronaphthalene-2-ol;
cis-6-phenyl-5-[4-(2-pyrrolidin-1-yl-ethoxy)-phenyl]-5,6,7,8-tetrahydronaphthalene-2-ol;
cis-1-[6'-pyrrolidinoethoxy-3'-pyridyl]-2-phenyl-6-hydroxy-1,2,3,4-tetrahydro aphthalene;
1-(4'-pyrrolidinoethoxyphenyl)-2-(4"-fluorophenyl)-6-hydroxy-1,2,3,4-tetrahydroisoquinoline;
cis-6-(4-hydroxyphenyl)-5-[4-(2-piperidin-1-yl-ethoxy)-phenyl]-5,6,7,8-tetrahydro-naphthalene-2-ol;
1-(4'-pyrrolidinoethoxyphenyl)-2-phenyl-6-hydroxy-1,2,3,4-tetrahydroisoquinoline and pharmaceutically acceptable salts thereof. An especially preferred salt of (-)-cis-6-phenyl-5-[4-(2-pyrrolidin-1-yl-ethoxy)-phenyl]-5,6,7,8-tetrahydro-naphthalene-2-ol is the D-tartrate salt.

The estrogen agonists / antagonists and/or testosterone, if applicable, can be administered in the form of pharmaceutically acceptable salts. The salts are conveniently formed, as is usual in organic chemistry, by reacting the compound of this invention with a suitable acid. The salts are formed usually in high yields at moderate temperatures, and often are prepared by merely isolating the compound from a suitable acidic wash as the final step of the synthesis. The salt-forming acid is dissolved in an appropriate organic solvent, or aqueous organic solvent, such as an alkanol, ketone or ester. On the other hand, if the compound of this invention is desired in the free base form, it is isolated from a basic final wash step, according to the usual practice. A preferred technique for preparing hydrochlorides is to dissolve the free base in a suitable solvent and dry the solution thoroughly, as over molecular sieves, before bubbling hydrogen chloride gas through it. A preferred salt of (-)-cis-6-phenyl-5-[4-(2-pyrrolidin-1-yl-ethoxy)-phenyl]-5,6, 7,8-tetrahydro-naphthalene-2-ol is the D-(-)-tartrate salt. It will also be recognized that it is possible to administer amorphous forms of the estrogen agonists / antagonists and/or testosterone, if applicable.

The expression "pharmaceutically acceptable salts" includes both pharmaceutically acceptable acid addition

salts and pharmaceutically acceptable cationic salts. The expression "pharmaceutically-acceptable cationic salts" is intended to define, but is not limited to, such salts as the alkali metal salts, (e.g. sodium and potassium), alkaline earth metal salts (e.g., calcium and magnesium), aluminum salts, ammonium salts, and salts with organic amines such as benzathine (N,N'-dibenzylethylenediamine), choline, diethanolamine, ethylenediamine, meglumine (N-methylglucamine), benethamine (N-benzylphenethylamine), diethylamine, piperazine, tromethamine (2-amino-2-hydroxymethyl-1,3-propanediol) and procaine. The expression "pharmaceutically-acceptable acid addition salts" is intended to define, but is not limited to, such salts as the hydrochloride, besylate, hydrobromide, sulfate, hydrogen sulfate, phosphate, hydrogen phosphate, dihydrogenphosphate, acetate, succinate, citrate, methanesulfonate (mesylate) and p-toluenesulfonate (tosylate) salts.

One of ordinary skill in the art will recognize that certain estrogen agonists / antagonists will contain one or more atoms which may be in a particular stereochemical, tautomeric, or geometric configuration, giving rise to stereoisomers, tautomers and configurational isomers. All such tautomers and isomers and mixtures thereof are included in this invention. Hydrates and solvates of the compounds of this invention are also included.

The subject invention also includes isotopically-labeled testosterone and/or estrogen agonists / antagonists, which are structurally identical, but for the fact that one or more atoms are replaced by an atom having an atomic mass or mass number different from the atomic mass or mass number usually found in nature. Examples of isotopes that can be incorporated into compounds of the invention include isotopes of hydrogen, carbon, nitrogen, oxygen, phosphorous, sulfur, fluorine and chlorine, such as $^{2}H$ , $^{3}H$, $^{13}C$, $^{14}C$, $^{15}N$, $^{18}O$, $^{17}O$, $^{31}P$, $^{32}P$, $^{35}S$, $^{18}F$ and $^{36}Cl$, respectively. Compounds of the present invention, prodrugs thereof, and pharmaceutically acceptable salts of said compounds and of said prodrugs which contain the aforementioned isotopes and/or other isotopes of other atoms are within the scope of this invention. Certain isotopically labeled compounds of the present invention, for example those into which radioactive isotopes such as $^{3}H$ and $^{14}C$ are incorporated, are useful in drug and/or substrate tissue distribution assays. Tritiated, i.e., $^{3}H$, and carbon-14, i.e., $^{14}C$, isotopes are particularly preferred for their ease of preparation and detectability. Further, substitution with heavier isotopes such as deuterium, i.e., $^{2}H$, may afford certain therapeutic advantages resulting from greater metabolic stability, for ∈ example increased *in vivo* half-life or reduced dosage requirements and, hence, may be preferred in some circumstances. Isotopically labeled compounds of this invention and prodrugs thereof can generally be prepared by carrying out known or referenced procedures and by substituting a readily available isotopically labeled reagent for a non-isotopically labeled reagent.

Those of ordinary skill in the art will recognize that physiologically active compounds that have accessible hydroxy groups can be administered in the form of pharmaceutically acceptable esters. The compounds of this invention can be effectively administered as an ester, formed on the hydroxy groups, just as one skilled in pharmaceutical chemistry would expect. It is possible, as has long been known in pharmaceutical chemistry, to adjust the rate or duration of action of the compound by appropriate choices of ester groups.

Certain ester groups are preferred when a compound of this invention contains an ester. The estrogen agonists / antagonists compounds of formula I and IA may contain ester groups at various positions as defined herein above, where these groups are represented as $-COOR^9$, $R^9$ is $C_1$-$C_{14}$ alkyl, $C_1$-$C_3$ chloroalkyl, $C_1$-$C_3$ fluoroalkyl, $C_5$-$C_7$ cycloalkyl, phenyl, or phenyl mono- or disubstituted with $C_1$-$C_4$ alkyl, $C_1$-$C_4$ alkoxy, hydroxy, nitro, chloro, fluoro or tri (chloro or fluoro)methyl.

Testosterone can be administered in the form of an ester. Examples of suitable esters include the propionate, enanthate, cypionate and undecanoate esters.

As used herein, the term " therapeutically effective amount" means an amount of a compound or combination of compounds that is capable of treating a described pathological condition. The specific dose of a compound or combination of compounds administered according to this invention will, of course, be determined by the particular circumstances surrounding the case including, for example, the compound administered, the route of administration, and the severity of the pathological condition being treated.

The dose of the compounds to be administered to a patient is rather widely variable and subject to the judgement of the attending physician. It should be noted that it may be necessary to adjust the dose of a compound when it is administered in the form of a salt, such as a laureate, the salt forming moiety of which has an appreciable molecular weight.

The following dosage amounts are for an average human patient having a weight of about 65 kg to about 70 kg. The skilled practitioner will readily be able to determine the dosage amount required for a patient whose weight falls outside the 65 kg to 70 kg range, based upon the medical history of the patient. All doses set forth herein are daily doses. Calculation of the dosage amount for forms other than the free base form, such as salts or hydrates, is easily accomplished by performing a simple ratio relative to the molecular weights of the forms involved.

The general range of effective administration rates of an estrogen agonist / antagonist is from about 0.001 mg/day to about 200 mg/day. A preferred rate range is from about 0.010 mg/day to about 100 mg/day. Of course, it is often practical to administer the daily dose of compound in portions, at various hours of the day. However, in any given case, the amount of compound administered will depend on such factors as the potency of the specific estrogen agonist/antagonist, the solubility of the compound, the formulation used and the route of administration.

Exogenous testosterone has been administered to males to increase testosterone levels. One important aspect of the present invention is that the combination of an estrogen agonist / antagonist with testosterone provides for decreased doses of testosterone when compared with the doses administered when testosterone is administered alone. In combination with an estrogen agonist / antagonist, it is possible to administer one third or even one half the dose of testosterone. Using a reduced dose of testosterone is beneficial because the administration of exogenous testosterone can suppress endogenous testosterone secretion due to negative feedback. Moreover, the estrogen agonist / antagonist also blocks the negative feedback by interfering with estrogen receptors in the brain. In addition, some testosterone is converted into estrogen in males. Estrogen in males has some undesired affects that are blocked by the administration of an estrogen agonist/ antagonist. Examples of a suitable dose range for testosterone when used in combination with an estrogen agonist / antagonist includes about 1 to about 10 mg/day for a transdermal dose or the equivalent dose depending on the manner of dosing. A preferred dose range is about 1 to about 4 mg/day.

Methods of formulation are well known in the art and are disclosed, for example, in Remington's Pharmaceutical Sciences, Mack Publishing Company, Easton, Pa., 19th Edition (1995). Pharmaceutical compositions for use within the present invention can be in the form of sterile, non-pyrogenic liquid solutions or suspensions, coated capsules, suppositories, lyophilized powders, transdermal patches or other forms known in the art.

Capsules are prepared by mixing the compound with a suitable diluent and filling the proper amount of the mixture in capsules. The usual diluents include inert powdered substances such as starch of many different kinds, powdered cellulose, especially crystalline and microcrystalline cellulose, sugars such as fructose, mannitol and sucrose, grain flours and similar edible powders.

Tablets are prepared by direct compression, by wet granulation, or by dry granulation. Their formulations usually incorporate diluents, binders, lubricants and disintegrators as well as the compound. Typical diluents include, for example, various types of starch, lactose, mannitol, kaolin, calcium phosphate or sulfate, inorganic salts such as sodium chloride and powdered sugar. Powdered cellulose derivatives are also useful. Typical tablet binders are substances such as starch, gelatin and sugars such as lactose, fructose, glucose and the like. Natural and synthetic gums are also convenient, including acacia, alginates, methylcellulose, polyvinylpyrrolidine and the like. Polyethylene glycol, ethylcellulose and waxes can also serve as binders.

A lubricant may be necessary in a tablet formulation to prevent the tablet and punches from sticking in the die. The lubricant is chosen from such slippery solids as talc, magnesium and calcium stearate, stearic acid and hydrogenated vegetable oils.

Tablet disintegrators are substances that facilitate the disintegration of a tablet to release a compound when the tablet becomes wet. They include starches, clays, celluloses, algins and gums, more particularly, corn and potato starches, methylcellulose, agar, bentonite, wood cellulose, powdered natural sponge, cation-exchange resins, alginic acid, guar gum, citrus pulp and carboxymethylcellulose, for example, may be used as well as sodium lauryl sulfate.

Tablets are often coated with sugar as a flavorant and sealant, or with film-forming protecting agents to modify the dissolution properties of the tablet. The compounds may also be formulated as chewable tablets, by using large amounts of pleasant-tasting substances such as mannitol in the formulation, as is now well-established in the art.

When it is desired to administer a compound as a suppository, the typical bases may be used. Cocoa butter is a traditional suppository base, which may be modified by addition of waxes to raise its melting point slightly. Water-miscible suppository bases comprising, particularly, polyethylene glycols of various molecular weights are in wide use.

The effect of the compounds may be delayed or prolonged by proper formulation. For example, a slowly soluble pellet of the compound may be prepared and incorporated in a tablet or capsule. The technique may be improved by making pellets of several different dissolution rates and filling capsules with a mixture of the pellets. Tablets or capsules may be coated with a film which resists dissolution for a predictable period of time. Topical formulations may be designed to yield delayed and/or prolonged percutaneous absorption of a compound. Even the parenteral preparations may be made long-acting, by dissolving or suspending the compound in oily or emulsified vehicles which allow it to disperse only slowly in the serum.

The term "prodrug" means a compound that is transformed *in vivo* to yield a compound of the present invention. The transformation may occur by various mechanisms, such as through hydrolysis in blood. A discussion of the use of prodrugs is provided by T. Higuchi and W. Stella, "Pro-drugs as Novel Delivery Systems," Vol. 14 of the A.C.S. Symposium Series, and in Bioreversible Carriers in Drug Design, ed. Edward B. Roche, American Pharmaceutical Association and Pergamon Press, 1987.

For example, if a compound of the present invention contains a carboxylic acid functional group, a prodrug can comprise an ester formed by the replacement of the hydrogen atom of the acid group with a group such as ($C_1$-$C_8$) alkyl, ($C_2$-$C_{12}$)alkanoyloxymethyl, 1-(alkanoyloxy)ethyl having from 4 to 9 carbon atoms, 1-methyl-1-(alkanoyloxy)-ethyl having from 5 to 10 carbon atoms, alkoxycarbonyloxymethyl having from 3 to 6 carbon atoms, 1-(alkoxycarbonyloxy)ethyl having from 4 to 7 carbon atoms, 1-methyl-1-(alkoxycarbonyloxy)ethyl having from 5 to 8 carbon atoms, N-(alkoxycarbonyl)aminomethyl having from 3 to 9 carbon atoms, 1-(N-(alkoxycarbonyl)amino)ethyl having from 4 to

10 carbon atoms, 3-phthalidyl, 4-crotonolactonyl, gamma-butyrolacton-4-yl, di-N,N-($C_1$-$C_2$)alkylamino($C_2$-$C_3$)alkyl (such as α-dimethylaminoethyl), carbamoyl-($C_1$-$C_2$)alkyl, N,N-di($C_1$-$C_2$)alkylcarbamoyl-($C_1$-$C_2$)alkyl and piperidino-, pyrrolidino- or morpholino($C_2$-$C_3$)alkyl.

Similarly, if a compound of the present invention comprises an alcohol functional group, a prodrug can be formed by the replacement of the hydrogen atom of the alcohol group with a group such as ($C_1$-$C_6$)alkanoyloxymethyl, 1-(($C_1$-$C_6$)alkanoyloxy)ethyl, 1-methyl-1-(($C_1$-$C_6$)alkanoyloxy)ethyl, ($C_1$-$C_6$)alkoxycarbonyloxymethyl, N-($C_1$-$C_6$)alkoxycarbonylaminomethyl, succinoyl, ($C_1$-$C_6$)alkanoyl, α-amino($C_1$-$C_4$)alkanoyl, arylacyl and α-aminoacyl, or α-aminoacyl-α-aminoacyl, where each α-aminoacyl group is independently selected from the naturally occurring L-amino acids, $P(O)(OH)_2$, -P(O)(O($C_1$-$C_6$)alkyl)$_2$ or glycosyl (the radical resulting from the removal of a hydroxyl group of the hemiacetal form of a carbohydrate).

If a compound of the present invention comprises an amine functional group, a prodrug can be formed by the replacement of a hydrogen atom in the amine group with a group such as $R^X$-carbonyl, $R^X$O-carbonyl, $NR^XR^{X'}$-carbonyl where $R^X$ and $R^{X'}$ are each independently ($C_1$-$C_{10}$)alkyl, ($C_3$-$C_7$)cycloalkyl, benzyl, or $R^X$-carbonyl is a natural α-aminoacyl or natural α-aminoacyl-natural α-aminoacyl, -C(OH)C(O)O$Y^X$ wherein $Y^X$ is H, ($C_1$-$C_6$)alkyl or benzyl), -C(O$Y^{X0}$) $Y^{X1}$ wherein $Y^{X0}$, is ($C_1$-$C_4$) alkyl and $Y^{X1}$ is ($C_1$-$C_6$)alkyl, carboxy($C_1$-$C_6$)alkyl, amino($C_1$-$C_4$)alkyl or mono-N- or di-N, N-($C_1$-$C_6$)alkylaminoalkyl, -C($Y^{X2}$) $Y^{X3}$ wherein $Y^{X2}$ is H or methyl and $Y^{X3}$ is mono-N- or di-N,N-($C_1$-$C_6$)alkylamino, morpholino, piperidin-1-yl or pyrrolidin-1-yl.

Advantageously, the present invention also provides kits for use by a consumer to treat andropause and the associated conditions. The kits comprise a) one or more pharmaceutical compositions comprising an estrogen agonist / antagonist, and/or testosterone, and a pharmaceutically acceptable carrier, vehicle or diluent; and b) instructions describing a method of using the pharmaceutical compositions to treat andropause or an associated condition, particularly for treating gynecomastia, lipid disorders, cardiovascular disease, atherosclerosis, hypogonadism, benign prostatic hyperplasia, or osteoporosis, or improving libido, or maintaining or improving vascular reactivity. The one or more pharmaceutical compositions contain an estrogen agonist /antagonist and testosterone. The estrogen agonist /antagonist can be in the same pharmaceutical composition as the testosterone, or the estrogen agonist / antagonist can be in a different pharmaceutical composition. An important aspect of the present invention is that both testosterone and an estrogen agonist /antagonist are administered to the patient. The kits can be configured in numerous way to accomplish this result.

A "kit" as used in the instant application includes a container for containing the pharmaceutical compositions and may also include divided containers such as a divided bottle or a divided foil packet. The container can be in any conventional shape or form as known in the art which is made of a pharmaceutically acceptable material, for example a paper or cardboard box, a glass or plastic bottle or jar, a resealable bag (for example, to hold a "refill" of tablets for placement into a different container), or a blister pack with individual doses for pressing out of the pack according to a therapeutic schedule. The container employed can depend on the exact dosage form involved, for example a conventional cardboard box would not generally be used to hold a liquid suspension. It is feasible that more than one container can be used together in a single package to market a single dosage form. For example, tablets may be contained in a bottle, which is in turn contained within a box.

An example of such a kit is a so-called blister pack. Blister packs are well known in the packaging industry and are being widely used for the packaging of pharmaceutical unit dosage forms (tablets, capsules, and the like). Blister packs generally consist of a sheet of relatively stiff material covered with a foil of a preferably transparent plastic material. During the packaging process, recesses are formed in the plastic foil. The recesses have the size and shape of individual tablets or capsules to be packed or may have the size and shape to accommodate multiple tablets and/ or capsules to be packed. Next, the tablets or capsules are placed in the recesses accordingly and the sheet of relatively stiff material is sealed against the plastic foil at the face of the foil which is opposite from the direction in which the recesses were formed. As a result, the tablets or capsules are individually sealed or collectively sealed, as desired, in the recesses between the plastic foil and the sheet. Preferably the strength of the sheet is such that the tablets or capsules can be removed from the blister pack by manually applying pressure on the recesses whereby an opening is formed in the sheet at the place of the recess. The tablet or capsule can then be removed via said opening.

It may be desirable to provide a written memory aid, where the written memory aid is of the type containing information and/or instructions for the physician, pharmacist or patient, e.g., in the form of numbers next to the tablets or capsules whereby the numbers correspond with the days of the regimen which the tablets or capsules so specified should be ingested or a card which contains the same type of information. Another example of such a memory aid is a calendar printed on the card e.g., as follows "First Week, Monday, Tuesday," ... etc .... "Second Week, Monday, Tuesday,..." etc. Other variations of memory aids will be readily apparent. A "daily dose" can be a single tablet or capsule or several tablets or capsules to be taken on a given day.

Another specific embodiment of a kit is a dispenser designed to dispense the daily doses one at a time. Preferably, the dispenser is equipped with a memory-aid, so as to further facilitate compliance with the regimen. An example of such a memory-aid is a mechanical counter which indicates the number of daily doses that has been

dispensed. Another example of such a memory-aid is a battery-powered micro-chip memory coupled with a liquid crystal readout, or audible reminder signal which, for example, reads out the date that the last daily dose has been taken and/or reminds one when the next dose is to be taken.

The kits of the present invention may also include, in addition to an estrogen agonist / antagonist and testosterone, one or more additional pharmaceutically active compounds. Preferably, the additional compound is another estrogen agonist / antagonist or another compound useful to treat andropause or an associated condition. The additional compounds may be administered in the same dosage form as the estrogen agonist / antagonist and/or testosterone or in different dosage forms. Likewise, the additional compounds can be administered at the same time as the estrogen agonist / antagonist and/or testosterone or at different times.

It is also noted that the estrogen agonist / antagonist and testosterone can be administered in the same dosage form or different dosage forms. Moreover, the estrogen agonist /antagonist and testosterone can be in the same pharmaceutical composition or in different pharmaceutical compositions and can be administered simultaneously or sequentially in any order. For example, the estrogen agonist /antagonist can be administered as a tablet and the testosterone administered transdermally by a patch that is placed on the skin.

All documents cited herein, including patents and patent applications, are hereby incorporated by reference. The protocols presented below are intended to illustrate particular embodiments of the invention and are not intended to limit the scope of the invention or the specification or claims in any manner.

Protocols

The estrogen agonist /antagonist and testosterone combinations of the present invention can be tested for relative efficacy and potency in the following procedures:

<u>Effect on Prostate Weight</u>

Male Sprague-Dawley rats, three months of age are administered by subcutaneous injection either vehicle (10% ethanol in water), estradiol (30 μg/kg), testosterone (1 mg/kg) or a combination of an estrogen agonist / antagonist and testosterone daily for 14 days. After 14 days the animals are sacrificed, the prostate is removed and the wet prostate weight is determined. Mean weight is determined and statistical significance ($p<0.05$) is determined compared to the vehicle-treated group using Student's t-test.

<u>Bone mineral density</u>

Bone mineral density, a measure of bone mineral content, accounts for greater than 80% of a bone's strength. Loss of bone mineral density with age and/or disease reduces a bone's strength and renders it more prone to fracture. Bone mineral content is accurately measured in people and animals by dual x-ray absorptiometry (DEXA) such that changes as little as 1% can be quantified. DEXA can be used to evaluate changes in bone mineral density.

<u>Male osteoporosis</u>

Adult orchidectomized (ORX) rats can be used as a model of male osteoporosis.

Fifty Sprague-Dawley male rats can be sham-operated or ORX at 10 months of age. A number of rats can be autopsied on day 0 to give basal controls. ORX rats are treated (daily p.o.) with either vehicle (10% ethanol in water) or a combination of an estrogen agonist /antagonist and testosterone for 60 days at various doses. All rats are given subcutaneous injections with 10 mg/kg of calcein (Sigma Chemical Co., St. Louis, MO) on 13 and 3 days before autopsy.

1) <u>Total serum cholesterol:</u> Total serum cholesterol is determined using a high performance cholesterol colormetic assay (Boehringer Mannheim Biochemicals, Indianapolis, IN).

2) <u>Prostate weight:</u> The prostate weight is determined immediately at autopsy.

3) <u>Femoral Bone Mineral Measurements:</u> The right femur from each rat is removed at autopsy and scanned using dual energy x-ray absorptiometry (DEXA, QDR 1000/W, Hologic Inc., Waltham, MA) equipped with "Regional High Resolution Scan" software (Hologic Inc., Waltham, MA). The scan field size is 5.08 x 1.902 cm, resolution is 0.0254 x 0.0127 cm and scan speed is 7.25 mim/second. The femoral scan images are analyzed and bone area, bone mineral content (BMC), and bone mineral density (BMD) of whole femora (WF), distal femoral metaphyses (DFM), and femoral shaft (FS) are determined according to the method described in H. Z. Ke et al., Droloxifene, a New Estrogen Antagonist/Agonist, Prevents Bone Loss in Ovariectomized Rats. *Endocrinology* 136;2435-2441, 1995.

4) <u>Third Lumbar Vertebral Body (LV3) Histomorphometry</u>: The LV3 can be removed at autopsy, dissected free of muscle, fixed in 70% ethanol, dehydrated in graded concentrations of ethanol, defatted in acetone, then embedded in methyl methacrylate (Eastman Organic Chemicals, Rochester, NY). Longitudinal sections of LV3 at 4 and 10 μm

thickness are cut using Reichert-Jung Polycut S microtome. One 4 μm and one 10 μm sections from each rat will be used for cancellous bone histomorphometry. The 4 μm sections are stained with modified Masson's Trichrome stain while the 10 μm section remain unstained.

A Bioquant OS/2 histomorphometry system (R&M Biometrics, Inc., Nashville, TN) is used for the static and dynamic histomorphometric measurements of the secondary spongiosa of the proximal tibial metaphyses between 1.2 and 3.6 mm distal to the growth plate-epiphyseal junction. The first 1.2 mm of the tibial metaphyseal region needs to be omitted in order to restrict measurements to the secondary spongiosa. The 4 μm sections will be used to determine indices related to bone volume, bone structure, and bone resorption, while the 10 μm sections will be used to determine indices related to bone formation and bone turnover.

(A). Measurements and calculations related to trabecular bone volume and structure:

1. Total metaphyseal area (TV, $mm^2$): metaphyseal area between 1.2 and 3.6 mm distal to the growth plate-epiphyseal junction.
2. Trabecular bone area (BV, $mm^2$): total area of trabeculae within TV.
3. Trabecular bone perimeter (BS, mm): the length of total perimeter of trabaculae.
4. Trabecular bone volume (BV/TV,%): BV / TV x 100.
5. Trabecular bone number (TBN,#/mm): 1.199 / 2 x BS / TV.
6. Trabecular bone thickness (TBT, μm): (2000 /1.199) x (BV / BS).
7. Trabecular bone separation (TBS, μm): (2000 x 1.199) x (TV - BV).

(B). Measurements and calculations related to bone resorption:

1. Osteoclast number (OCN, #): total number of osteoclast within total metaphyseal area.
2. Trabecular bone area (OCP, mm): length of trabecular perimeter covered by osteoclast.
3. Osteoclast number/mm (OCN/mm, #/mm): OCN / BS.
4. Percent osteoclast perimeter (%OCP,%): OCP / BS x 100.

(C). Measurements and calculations related to bone formation and turnover:

1. Single-calcein labeled perimeter (SLS, mm): total length of trabecular perimeter labeled with one calcein label.
2. Double-calcein labeled perimeter (DLS, mm): total length of trabecular perimeter labeled with two calcein labels.
3. Inter-labeled width (ILW, μm): average distance between two calcein labels.
4. Percent mineralizing perimeter (PMS,%):(SLS/2 + DLS) / BS x 100.
5. Mineral apposition rate (MAR, μm/day): ILW / label interval.
6. Bone formation rate/surface ref. (BFR/BS, $\mu m^2/d/\mu m$): (SLS/2 + DLS) x MAR / BS.
5. Bone turnover rate (BTR, %/y): (SLS/2 + DLS) x MAR / BV x 100.

5) Compression test of fifth lumbar vertebral body. Using a Material Testing System (Model 810, MTS systems Corp., Minneapolis, MN), mechanical testings are performed on the fifth lumbar vertebral body (LV5). The load displacement curve is obtained from each test. A compression test is used to determine the mechanical properties of LV5, as described by Mosekilde et al. (Mosekilde, L., et al., Endocrinol 1994; 134:2126-2134). LV5 (with the two epiphyseal ends, posterior pedicle arch and spinous process removed) is compressed to failure at a displacement rate of 0.1 mm/second using a 2.5 kN load cell (MTS model 661, 14A-03). The maximal load and stiffness are calculated from the load-displacement curve.

Effect on total cholesterol levels

The effect of the combination of an estrogen agonist /antagonist and testosterone on plasma levels of total cholesterol is measured in the following way. Blood samples are collected via cardiac puncture from anesthetized (S-D) rats 4-6 months of age that are treated with the combination (10-1000 μg/kg/day, for example, sc or orally for 28 days or with vehicle for the same time), or sham operated. The blood is placed in a tube containing 30μL of 5% EDTA (10μL

EDTA/1 mL of blood). After centrifugation at 2500 rpm for 10 minutes at 20°C the plasma is removed and stored at -20°C unit assay. The total cholesterol is assayed using a standard enzymatic determination kit from Sigma Diagnostics.

Brachial Artery Reactivity

A primary outcome for a clinical study will be the change in endothelial-dependent vasodilator capacity in the brachial artery following 8 weeks of treatment with an estrogen agonist / antagonist and testosterone or placebo. The vasodilator stimulus used will be an increase in brachial artery flow caused by ischemic hyperemia in the distal limb. The changes in diameter of the brachial artery can be imaged using high resolution 2-D ultrasound with the measurement of change in diameter being based on image processing techniques specifically designed to measure diameter of the brachial artery using automated boundary detection algorithms. Through the use of standardized protocols for subject preparation, image acquisition and image analysis, accurate and precise measurement of brachial artery diameter and wall thickness have been developed, validated and employed in numerous clinical studies.

Participants are allowed to rest in the supine position for 10 minutes in a quiet room. A blood pressure cuff is placed on the right forearm just below the antecubital fossa and the arm is supported with sand bags to allow inflation and deflation of the blood pressure cuff within movement of the arm. The blood pressure and heart rate are measured in the left arm using an automated sphygmomanometer. Once a comfortable and secure position has been established and the blood pressure is determined, images of the brachial artery at baseline are obtained (see section entitled "Image Acquisition"). After baseline imaging, the blood pressure cuff is rapidly inflated to 30 mm Hg greater than the systolic blood pressure for 5 minutes. The brachial artery is imaged again starting 30 seconds prior to cuff release and continuing for a total of 3 minutes following cuff release.

Image Acquisition

The right brachial artery is examined approximately 7 cm proximal to the bend of the elbow using a high resolution ultrasound system. A brief doppler signal is recorded in the vessel to confirm identification. Once the near and far wall boundaries are visualized with careful transducer movements, the transducer is maintained at this location throughout the examination. Careful observation of surrounding tissues provide internal landmarks to confirm that this is accomplished. Baseline images are then recorded for approximately 2 minutes on a video recorder. During the 5-minute interval during which the right blood pressure cuff is inflated to 30 mmHg above systolic pressure, the sonographer alternately views the B-mode image and the doppler signal to confirm that a high quality image is being maintained and that a significant modification of blood flow is being achieved in the vessel. During the final 30 seconds prior to rapid cuff deflation, high quality B-mode images are recorded. Immediately after cuff release, doppler signals are recorded for 10-15 seconds to observe the peak flow after cuff release, after which high quality B-mode images are continuously recorded for 3 minutes.

Image Analysis

The videotape is completely reviewed by the image analysis technicians prior to analysis. After identifying the portion of the tape demonstrating the brachial artery at baseline, 30 frames are digitized with a frame grabber into 512 x 512 x 8 bit grey scales and stored on the image analysis computer. Using a semi-automated boundary detection algorithm, the medial-advenitial boundary on the near and far wall of the brachial artery is located over an arterial segment 2.0-2.5 cm in length. If a boundary point is obviously displaced from the true location of the medial-adventitial boundary, then the image analysis technician will manually edit the boundary point in question. However, every effort is made to minimize the editing used. The average diameter of the artery is automatically calculated and the mean diameter from the 3-D baseline frames is used to determine the baseline diameter. The exact same procedure is repeated to determine the diameter of the artery just prior to cuff release. Similar methods are used to determine the maximum diameter that occurs during the 3 minutes immediately following cuff release. Time from cuff release to point of maximum dilation will also be recorded.

Primary and Secondary Outcome Measures

The primary outcome measure is relative change in mean arterial diameter calculated as follows:

$$\frac{\text{max diameter}}{\text{baseline diameter}} \times 100.$$

Time to maximum dilation and percent change from end of cuff occlusion to maximum dilation will also be determined.

## Claims

**1.** The use of a therapeutically effective amount of an estrogen agonist / antagonist compound of formula I

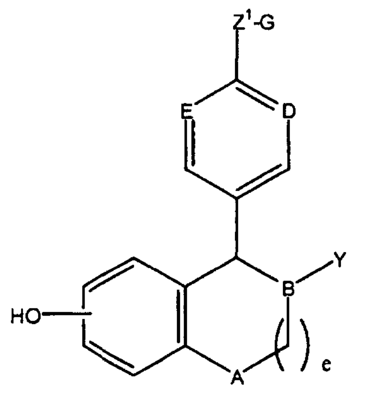

(I)

wherein:

A is selected from $CH_2$ and NR;
B, D and E are independently selected from CH and N;
Y is

(a) phenyl, optionally substituted with 1-3 substituents independently selected from $R^4$;
(b) naphthyl, optionally substituted with 1-3 substituents independently selected from $R^4$;
(c) $C_3$-$C_8$ cycloalkyl, optionally substituted with 1-2 substituents independently selected from $R^4$;
(d) $C_3$-$C_8$ cycloalkenyl, optionally substituted with 1-2 substituents independently selected from $R^4$;
(e) a five membered heterocycle containing up to two heteroatoms selected from the group consisting of -O-, -$NR^2$- and -$S(O)_n$-, optionally substituted with 1-3 substituents independently selected from $R^4$;
(f) a six membered heterocycle containing up to two heteroatoms selected from the group consisting of -O-, -$NR^2$- and -$S(O)_n$- optionally substituted with 1-3 substituents independently selected from $R^4$; or
(g) a bicyclic ring system consisting of a five or six membered heterocyclic ring fused to a phenyl ring, said heterocyclic ring containing up to two heteroatoms selected from the group consisting of -O-, -$NR^2$- and -$S(O)_n$-, optionally substituted with 1-3 substituents independently selected from $R^4$;

$Z^1$ is

(a) $-(CH_2)_p W(CH_2)_q-$;
(b) $-O(CH_2)_p CR^5R^6-$;
(c) $-O(CH_2)_pW(CH_2)_q-$;
(d) $-OCHR^2CHR^3-$; or
(e) $-SCHR^2CHR^3-$;

G is

(a) $-NR^7R^8$;
(b)

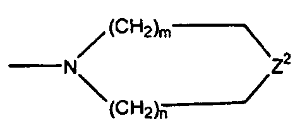

wherein n is 0, 1 or 2; m is 1, 2 or 3; $Z^2$ is -NH-, -O-, -S-, or $-CH_2-$; optionally fused on adjacent carbon atoms with one or two phenyl rings and, optionally independently substituted on carbon with one to three substituents and, optionally, independently on nitrogen with a chemically suitable substituent selected from $R^4$; or

(c) a bicyclic amine containing five to twelve carbon atoms, either bridged or fused and optionally substituted with 1-3 substituents independently selected from $R^4$; or

$Z^1$ and G in combination may be

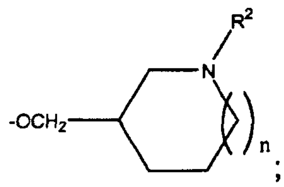

Wis

(a) $-CH_2-$;
(b) -CH=CH-;
(c) -O-;
(d) $-NR^2-$;
(e) $-S(O)_n-$;
(f)

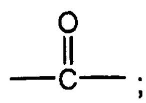

(g) $-CR^2(OH)-$;
(h) $-CONR^2-$;
(i) $-NR^2CO-$;
(j)

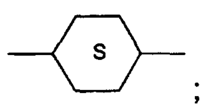

or
(k) $-C\equiv C-$;

R is hydrogen or $C_1$-$C_6$ alkyl;
$R^2$ and $R^3$ are independently

(a) hydrogen; or
(b) $C_1$-$C_4$ alkyl;

$R^4$ is

(a) hydrogen;
(b) halogen;
(c) $C_1$-$C_6$ alkyl;
(d) $C_1$-$C_4$ alkoxy;
(e) $C_1$-$C_4$ acyloxy;

(f) $C_1$-$C_4$ alkylthio;
(g) $C_1$-$C_4$ alkylsulfinyl;
(h) $C_1$-$C_4$ alkylsulfonyl;
(i) hydroxy ($C_1$-$C_4$)alkyl;
(j) aryl ($C_1$-$C_4$)alkyl;
(k) $-CO_2H$;
(l) -CN;
(m) -CONHOR;
(n) $-SO_2NHR$;
(o) $-NH_2$;
(p) $C_1$-$C_4$ alkylamino;
(q) $C_1$-$C_4$ dialkylamino;
(r) $-NHSO_2R$;
(s) $-NO_2$;
(t) -aryl; or
(u) -OH;

$R^5$ and $R^6$ are independently $C_1$-$C_8$ alkyl or together form a $C_3$-$C_{10}$ carbocyclic ring;
$R^7$ and $R^8$ are independently

(a) phenyl;
(b) a $C_3$-$C_{10}$ carbocyclic ring, saturated or unsaturated;
(c) a $C_3$-$C_{10}$ heterocyclic ring containing up to two heteroatoms, selected from -O-, -N- and -S-;
(d) H;
(e) $C_1$-$C_6$ alkyl; or
(f) form a 3 to 8 membered nitrogen containing ring with $R^5$ or $R^6$;

$R^7$ and $R^8$ in either linear or ring form may optionally be substituted with up to three substituents independently selected from $C_1$-$C_6$ alkyl, halogen, alkoxy, hydroxy and carboxy;
a ring formed by $R^7$ and $R^8$ may be optionally fused to a phenyl ring;
e is 0, 1 or 2;
m is 1, 2 or 3;
n is 0, 1 or 2;
p is 0, 1, 2 or 3;
q is 0, 1, 2 or 3;

or an optical or geometric isomer thereof; or a pharmaceutically acceptable salt, N-oxide, ester, quaternary ammonium salt or prodrug thereof, and testosterone for the manufacture of a medicament for treating a decline in the level of the hormone testosterone in a male patient.

**2.** A use according to claim 1 wherein the estrogen agonist / antagonist is a compound of formula (IA)

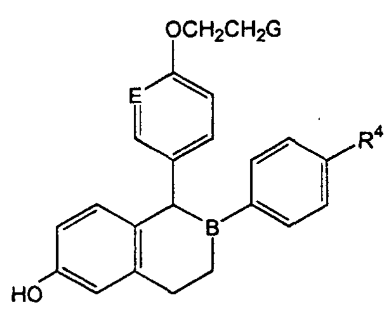

(IA)

wherein G is

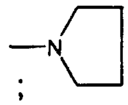 ; , 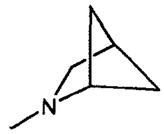 or 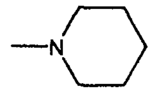

$R^4$ is H, OH, F, or Cl; and B and E are independently selected from CH and N or an optical or geometric isomer thereof; or a pharmaceutically acceptable salt, N-oxide, ester, quaternary ammonium salt, or a prodrug thereof.

**3.** A use according to claims 1 or 2 wherein the estrogen agonist / antagonist is (-)-cis-6-phenyl-5-[4-(2-pyrrolidin-1-yl-ethoxy)-phenyl]-5,6,7,8-tetrahydronaphthalene-2-ol or an optical or geometric isomer thereof; a pharmaceutically acceptable salt, N-oxide, ester, quaternary ammonium salt, or a prodrug thereof.

**4.** A use according to claim 3 wherein the estrogen agonist / antagonist is in the form of a D-tartrate salt.

**5.** The use of a therapeutically effective amount of an estrogen agonist / antagonist compound of formula I

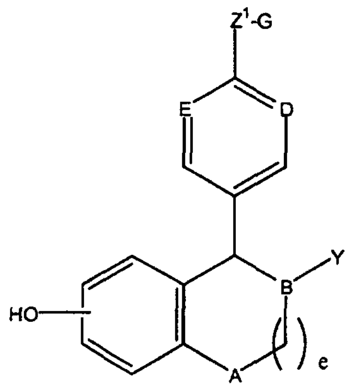

(I)

wherein:

A is selected from $CH_2$ and NR;
B, D and E are independently selected from CH and N;
Y is

(a) phenyl, optionally substituted with 1-3 substituents independently selected from $R^4$;
(b) naphthyl, optionally substituted with 1-3 substituents independently selected from $R^4$;
(c) $C_3$-$C_8$ cycloalkyl, optionally substituted with 1-2 substituents independently selected from $R^4$;
(d) $C_3$-$C_8$ cycloalkenyl, optionally substituted with 1-2 substituents independently selected from $R^4$;
(e) a five membered heterocycle containing up to two heteroatoms selected from the group consisting of -O-, $-NR^2-$ and $-S(O)_n-$, optionally substituted with 1-3 substituents independently selected from $R^4$;
(f) a six membered heterocycle containing up to two heteroatoms selected from the group consisting of -O-, $-NR^2-$ and $-S(O)_n-$ optionally substituted with 1-3 substituents independently selected from $R^4$; or
(g) a bicyclic ring system consisting of a five or six membered heterocyclic ring fused to a phenyl ring, said heterocyclic ring containing up to two heteroatoms selected from the group consisting of -O-, $-NR^2-$ and $-S(O)_n-$, optionally substituted with 1-3 substituents independently selected from $R^4$;

$Z^1$ is

(a) $-(CH_2)_p W(CH_2)_q-$;
(b) $-O(CH_2)_p CR^5R^6-$;

(c) $-O(CH_2)_pW(CH_2)_q-$;
(d) $-OCHR^2CHR^3-$; or
(e) $-SCHR^2CHR^3-$;

G is

(a) $-NR^7R^8$ ;
(b)

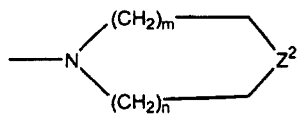

wherein n is 0, 1 or 2; m is 1, 2 or 3; $Z^2$ is -NH-, -O-, -S-, or $-CH_2-$; optionally fused on adjacent carbon atoms with one or two phenyl rings and, optionally independently substituted on carbon with one to three substituents and, optionally, independently on nitrogen with a chemically suitable substituent selected from $R^4$; or
(c) a bicyclic amine containing five to twelve carbon atoms, either bridged or fused and optionally substituted with 1-3 substituents independently selected from $R^4$; or

$Z^1$ and G in combination may be

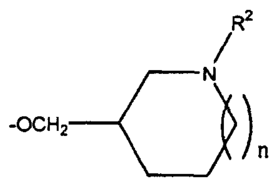
;

W is

(a) $-CH_2-$;
(b) -CH=CH-;
(c) -O-;
(d) $-NR^2-$;
(e) $-S(O)_n-$;
(f)

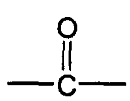
;

(g) $-CR^2(OH)-$;
(h) $-CONR^2-$;
(i) $-NR^2CO-$;
(j)

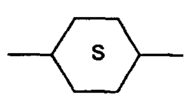
;

or
(k) -C≡C-;

R is hydrogen or $C_1$-$C_6$ alkyl;
$R^2$ and $R^3$ are independently

(a) hydrogen; or
(b) $C_1$-$C_4$ alkyl;

$R^4$ is

(a) hydrogen;
(b) halogen;
(c) $C_1$-$C_6$ alkyl;
(d) $C_1$-$C_4$ alkoxy;
(e) $C_1$-$C_4$ acyloxy;
(f) $C_1$-$C_4$ alkylthio;
(g) $C_1$-$C_4$ alkylsulfinyl;
(h) $C_1$-$C_4$ alkylsulfonyl;
(i) hydroxy ($C_1$-$C_4$)alkyl;
(j) aryl ($C_1$-$C_4$)alkyl;
(k) -$CO_2H$;
(l) -CN;
(m) -CONHOR;
(n) -$SO_2NHR$;
(o) -$NH_2$;
(p) $C_1$-$C_4$ alkylamino;
(q) $C_1$-$C_4$ dialkylamino;
(r) -$NHSO_2R$;
(s) -$NO_2$;
(t) -aryl; or
(u) -OH;

$R^5$ and $R^6$ are independently $C_1$-$C_8$ alkyl or together form a $C_3$-$C_{10}$ carbocyclic ring;
$R^7$ and $R^8$ are independently

(a) phenyl;
(b) a $C_3$-$C_{10}$ carbocyclic ring, saturated or unsaturated;
(c) a $C_3$-$C_{10}$ heterocyclic ring containing up to two heteroatoms, selected from -O-, -N- and -S-;
(d) H;
(e) $C_1$-$C_6$ alkyl; or
(f) form a 3 to 8 membered nitrogen containing ring with $R^5$ or $R^6$;

$R^7$ and $R^8$ in either linear or ring form may optionally be substituted with up to three substituents independently selected from $C_1$-$C_6$ alkyl, halogen, alkoxy, hydroxy and carboxy;
a ring formed by $R^7$ and $R^8$ may be optionally fused to a phenyl ring;
e is 0, 1 or 2;
m is 1, 2 or 3;
n is 0, 1 or 2;
p is 0, 1, 2 or 3;
q is 0, 1, 2 or 3;

or an optical or geometric isomer thereof; or a pharmaceutically acceptable salt, N-oxide, ester, quaternary ammonium salt or prodrug thereof, for the manufacture of a medicament for treating gynecomastia, lipid disorders, cardiovascular disease, atherosclerosis, hypogonadism, benign prostatic hyperplasia, osteoporosis, decreased libido, or decreased vascular reactivity in a male patient.

**6.** A use according to claim 5 wherein the estrogen agonist / antagonist is a compound of formula (IA)

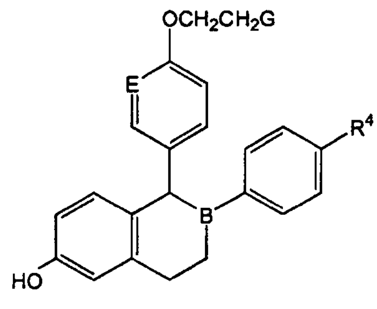

(IA)

wherein G is

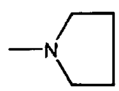 , 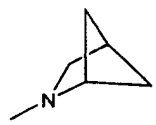 or 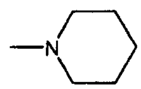 ;

$R^4$ is H, OH, F, or Cl; and B and E are independently selected from CH and N or an optical or geometric isomer thereof; or a pharmaceutically acceptable salt, N-oxide, ester, quaternary ammonium salt, or a prodrug thereof.

**7.** A use according to claims 5 or 6 wherein the estrogen agonist / antagonist is (- )-cis-6-phenyl-5-[4-(2-pyrrolidin-1-yl-ethoxy)-phenyl]-5,6,7 ,8-tetrahydronaphthalene-2-ol or an optical or geometric isomer thereof; a pharmaceutically acceptable salt, N-oxide, ester, quaternary ammonium salt, or a prodrug thereof.

**8.** A use according to claim 7 wherein the estrogen agonist / antagonist is in the form of a D-tartrate salt.

**9.** A kit for treating a decline in the level of the hormone testosterone in a male patient, gynecomastia, lipid disorders, cardiovascular disease, atherosclerosis, hypogonadism, benign prostatic hyperplasia, osteoporosis, decreased libido, or decreased vascular reactivity in a male patient, the kit comprising: one or more pharmaceutical compositions comprising an estrogen agonist / antagonist and testosterone; and wherein the estrogen agonists/ antagonist is a compound of formula I

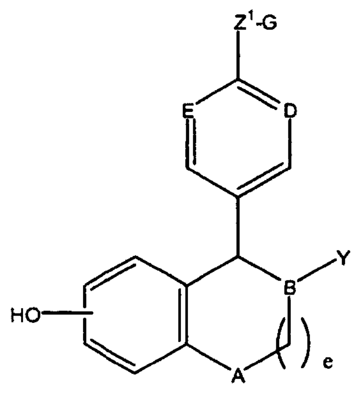

(I)

wherein:

A is selected from $CH_2$ and NR;
B, D and E are independently selected from CH and N;
Y is

(a) phenyl, optionally substituted with 1-3 substituents independently selected from $R^4$;
(b) naphthyl, optionally substituted with 1-3 substituents independently selected from $R^4$;
(c) $C_3$-$C_8$ cycloalkyl, optionally substituted with 1-2 substituents independently selected from $R^4$;
(d) $C_3$-$C_8$ cycloalkenyl, optionally substituted with 1-2 substituents independently selected from $R^4$;
(e) a five membered heterocycle containing up to two heteroatoms selected from the group consisting of -O-, -$NR^2$- and -$S(O)_n$-, optionally substituted with 1-3 substituents independently selected from $R^4$;
(f) a six membered heterocycle containing up to two heteroatoms selected from the group consisting of -O-, -$NR^2$- and -$S(O)_n$- optionally substituted with 1-3 substituents independently selected from $R^4$; or
(g) a bicyclic ring system consisting of a five or six membered heterocyclic ring fused to a phenyl ring, said heterocyclic ring containing up to two heteroatoms selected from the group consisting of -O-, -$NR^2$- and -$S(O)_n$-, optionally substituted with 1-3 substituents independently selected from $R^4$;

$Z^1$ is

(a) $-(CH_2)_p W(CH_2)_q-$;
(b) $-O(CH_2)_p CR^5R^6-$;
(c) $-O(CH_2)_pW(CH_2)_q-$;
(d) $-OCHR^2CHR^3-$; or
(e) $-SCHR^2CHR^3-$;

G is

(a) $-NR^7R^8$;
(b)

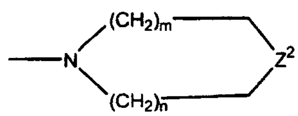

wherein n is 0, 1 or 2; m is 1, 2 or 3; $Z^2$ is -NH-, -O-, -S-, or $-CH_2-$; optionally fused on adjacent carbon atoms with one or two phenyl rings and, optionally independently substituted on carbon with one to three substituents and, optionally, independently on nitrogen with a chemically suitable substituent selected from $R^4$; or
(c) a bicyclic amine containing five to twelve carbon atoms, either bridged or fused and optionally substituted with 1-3 substituents independently selected from $R^4$; or

$Z^1$ and G in combination may be

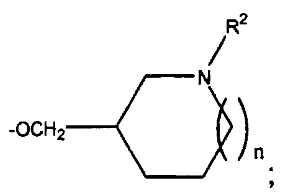

W is

(a) $-CH_2-$;
(b) -CH=CH-;
(c) -O-;
(d) $-NR^2-$;
(e) $-S(O)_n-$;
(f)

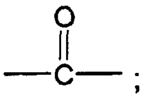

(g) $-CR^2(OH)-$;
(h) $-CONR^2-$;
(i) $-NR^2CO-$;
(j)

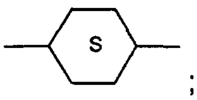

or
(k) -C≡C-;

R is hydrogen or $C_1$-$C_6$ alkyl;
$R^2$ and $R^3$ are independently

(a) hydrogen; or
(b) $C_1$-$C_4$ alkyl;

$R^4$ is

(a) hydrogen;
(b) halogen;
(c) $C_1$-$C_6$ alkyl;
(d) $C_1$-$C_4$ alkoxy;
(e) $C_1$-$C_4$ acyloxy;
(f) $C_1$-$C_4$ alkylthio;
(g) $C_1$-$C_4$ alkylsulfinyl;
(h) $C_1$-$C_4$ alkylsulfonyl;
(i) hydroxy ($C_1$-$C_4$)alkyl;
(j) aryl ($C_1$-$C_4$)alkyl;
(k) $-CO_2H$;
(l) -CN;
(m) -CONHOR;
(n) $-SO_2NHR$;
(o) $-NH_2$;
(p) $C_1$-$C_4$ alkylamino;
(q) $C_1$-$C_4$ dialkylamino;
(r) $-NHSO_2R$;
(s) $-NO_2$;
(t) -aryl; or
(u) -OH;

$R^5$ and $R^6$ are independently $C_1$-$C_8$ alkyl or together form a $C_3$-$C_{10}$ carbocyclic ring;
$R^7$ and $R^8$ are independently

(a) phenyl;
(b) a $C_3$-$C_{10}$ carbocyclic ring, saturated or unsaturated;
(c) a $C_3$-$C_{10}$ heterocyclic ring containing up to two heteroatoms, selected from -O-, -N- and -S-;
(d) H;
(e) $C_1$-$C_6$ alkyl; or
(f) form a 3 to 8 membered nitrogen containing ring with $R^5$ or $R^6$;

$R^7$ and $R^8$ in either linear or ring form may optionally be substituted with up to three substituents independently selected from $C_1$-$C_6$ alkyl, halogen, alkoxy, hydroxy and carboxy;
a ring formed by $R^7$ and $R^8$ may be optionally fused to a phenyl ring;
e is 0, 1 or 2;
m is 1, 2 or 3;
n is 0, 1 or 2;
p is 0, 1, 2 or 3;
q is 0, 1,2 or 3;

or an optical or geometric isomer thereof; or a pharmaceutically acceptable salt, N-oxide, ester, quaternary ammonium salt or prodrug thereof.

**10.** A kit according to claim 9 wherein the estrogen agonist / antagonist is a compound of formula (IA)

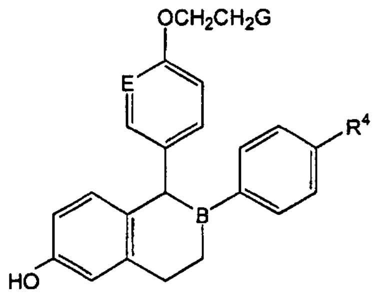

wherein G is

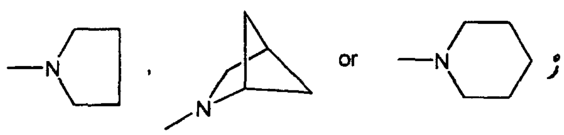

$R^4$ is H, OH, F, or Cl; and B and E are independently selected from CH and N or an optical or geometric isomer thereof; or a pharmaceutically acceptable salt, N-oxide, ester, quaternary ammonium salt, or a prodrug thereof.

**11.** A kit according to claims 9 or 10 wherein the estrogen agonist / antagonist is (-)-cis-6-phenyl-5-[4-(2-pyrrolidin-1 -yl-ethoxy)-phenyl]-5,6,7,8-tetrahydronaphthalene-2-ol or an optical or geometric isomer thereof; a pharmaceutically acceptable salt, N-oxide, ester, quaternary ammonium salt, or a prodrug thereof.

**12.** A kit according to claim 11 wherein the estrogen agonist / antagonist is in the form of a D-tartrate salt.

**13.** A combination of a compound of formula I, as defined in claim 1, or an optical or geometric isomer thereof; or a pharmaceutically acceptable salt, N-oxide, ester, quaternary ammonium salt or prodrug thereof and testosterone for use as an agent for treating testosterone deficiency in a male patient.

**14.** Pharmaceutical products containing a compound of formula I, as defined in claim 1, or an optical or geometric isomer thereof; or a pharmaceutically acceptable salt, N-oxide, ester, quaternary ammonium salt or prodrug thereof and testosterone as a combined preparation for simultaneous, separate or sequential use in treating testosterone deficiency in a male patient.

**Patentansprüche**

**1.** Verwendung einer therapeutisch wirksamen Menge einer Östrogenagonist- / -antagonistverbindung der Formel I

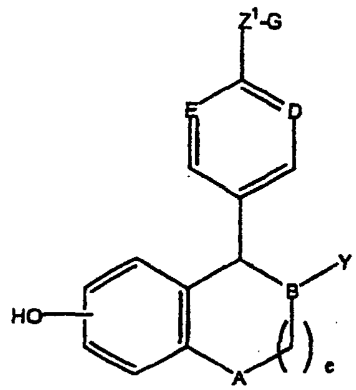

(I)

wobei gilt:

A ist aus $CH_2$ und NR ausgewählt;
B, D und E sind unabhängig aus CH und N ausgewählt ;
Y ist

(a) ein optional mit 1-3 unabhängig aus $R^4$ ausgewählten Substituenten substituiertes Phenyl;
(b) ein optional mit 1-3 unabhängig aus $R^4$ ausgewählten Substituenten substituiertes Naphthyl;
(c) ein optional mit 1-2 unabhängig aus $R^4$ ausgewählten Substituenten substituiertes $C_3$-$C_8$-Cycloalkyl;
(d) ein optional mit 1-2 unabhängig aus $R^4$ ausgewählten Substituenten substituiertes $C_3$-$C_8$-Cycloalkenyl;
(e) ein fünfgliedriger, optional mit 1-3 unabhängig aus $R^4$ ausgewählten Substituenten substituierter Heterozyklus, welcher bis zu zwei aus der aus -O-, -$NR^2$- und -$S(O)_n$- bestehenden Gruppe ausgewählte Heteroatome aufweist;
(f) ein sechsgliedriger, optional mit 1-3 unabhängig aus $R^4$ ausgewählten Substituenten substituierter Heterozyklus, welcher bis zu zwei aus der aus -O-, -$NR^2$- und -$S(O)_n$- bestehenden Gruppe ausgewählte Heteroatome aufweist; oder
(g) ein aus einem fünf- oder sechsgliedrigen, mit einem Phenylring kondensierten heterozyklischen Ring bestehendes bizyklisches Ringsystem, wobei besagter heterozyklischer Ring bis zu zwei aus der aus -O-, -$NR^2$- und -$S(O)_n$- bestehenden Gruppe ausgewählte Heteroatome aufweist, das optional mit 1-3 unabhängig aus $R^4$ ausgewählten Substituenten substituiert ist;

$Z^1$ ist

(a) -$(CH_2)_pW(CH_2)_q$-;
(b) -$O(CH_2)_pCR^5R^6$-;
(c) -$O(CH_2)_pW(CH_2)_q$-;
(d) -$OCHR^2CHR^3$-; oder
(e) -$SCHR^2CHR^3$-;

G ist

(a) -$NR^7R^8$;
(b)

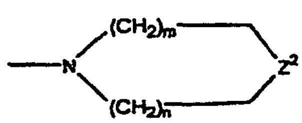

wobei n 0, 1 oder 2 ist; m 1, 2 oder 3 ist; $Z^2$ -NH-, -O-, -S- oder -$CH_2$- ist; optional an benachbarten

Kohlenstoffatomen mit einem oder zwei Phenylringen kondensiert und optional am Kohlenstoff unabhängig substituiert mit einem bis drei Substituenten und optional am Stickstoff unabhängig substituiert mit einem chemisch geeigneten Substituenten, ausgewählt aus $R^4$; oder
(c) ein fünf bis zwölf Kohlenstoffatome enthaltendes bizyklisches Amin, das entweder verbrückt oder kondensiert ist und optional mit 1-3 unabhängig aus $R^4$ ausgewählten Substituenten substituiert ist; oder

$Z^1$ und G in Kombination können sein

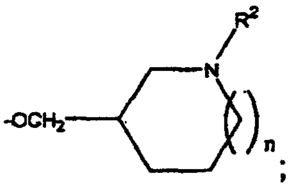

W ist

(a) $-CH_2-$;
(b) $-CH=C_H-$;
(c) -O-;
(d) $-NR^2-$;
(e) $-S(O)_n-$;
(f)

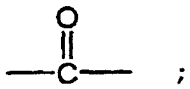

(g) $-CR^2$ (OH)- ;
(h) $-CONR^2$;
(i) $-NR^2CO-$;
(j)

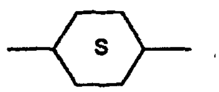

oder
(k) -C≡C- ;

R ist Wasserstoff oder $C_1$-$C_6$-Alkyl;
$R^2$ und $R^3$ sind unabhängig

(a) Wasserstoff; oder
(b) $C_1$-$C_4$-Alkyl;

$R^4$ ist

(a) Wasserstoff;
(b) Halogen;
(c) $C_1$-$C_6$-Alkyl ;
(d) $C_1$-$C_4$-Alkoxy ;
(e) $C_1$-$C_4$-Acyloxy ;
(f) $C_1$-$C_4$-Alkylthio ;
(g) $C_1$-$C_4$-Alkylsulfinyl ;

(h) $C_1$-$C_4$-Alkylsulfonyl ;
(i) Hydroxy($C_1$-$C_4$)Alkyl ;
(j) Aryl($C_1$-$C_4$)Alkyl ;
(k) -$CO_2H$ ;
(l) -CN;
(m) -CONHOR;
(n) -$SO_2NHR$;
(o) -$NH_2$ ;
(p) $C_1$-$C_4$-Alkylamino ;
(q) $C_1$-$C_4$-Dialkylamino;
(r) -$NHSO_2R$;
(s) -$NO_2$;
(t) -Aryl; oder
(u) -OH;

$R^5$ und $R^6$ sind unabhängig $C_1$-$C_8$-Alkyl order bilden zusammen einen $C_3$-$C_{10}$-carbozyklischen Ring;
$R^7$ und $R^8$ sind unabhängig

(a) Phenyl;
(b) ein gesättigter oder ungesättigter $C_3$-$C_{10}$carbozyklischer Ring;
(c) ein bis zu zwei aus -O-, -N- und -S- ausgewählte Heteroatome enthaltender $C_3$-$C_{10}$-heterozyklischer Ring;
(d) H;
(e) $C_1$-$C_6$-Alkyl; oder
(f) bilden mit $R^5$ oder $R^6$ einen 3- bis 8-gliedrigen, Stickstoff enthaltenden Ring;

$R^7$ und $R^8$ entweder als lineare Form oder Ringform können optional mit bis zu drei unabhängig aus $C_1$-$C_6$-Alkyl, Halogen, Alkoxy, Hydroxy und Carboxy ausgewählten Substituenten substituiert sein;
ein durch $R^7$ und $R^8$ gebildeter Ring ist optional mit einem Phenylring kondensiert;
e ist 0, 1 oder 2;
m ist 1,2 oder 3;
n ist 0, 1 oder 2;
p ist 0, 1, 2, oder 3;
q ist 0, 1, 2 oder 3;

oder eines optischen oder geometrischen Isomers davon; oder eines pharmazeutisch annehmbaren Salzes, N-Oxids, Esters, quaternären Ammoniumsalzes oder Prodrugs davon, und Testosteron zur Herstellung eines Medikamentes zur Behandlung einer Abnahme des Spiegels des Hormons Testosteron in einem männlichen Patienten.

**2.** Verwendung nach Anspruch 1, wobei der Östrogenagonist / - antagonist ist:

eine Verbindung der Formel (IA),

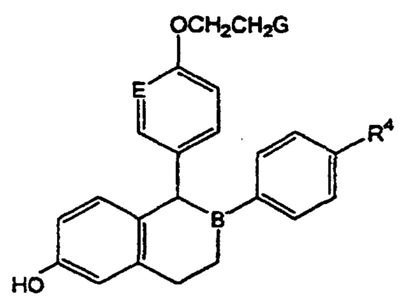

(IA)

wobei gilt:

G ist

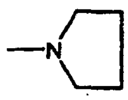 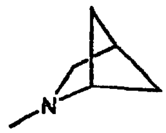 oder 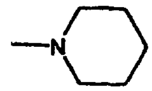 ;

$R^4$ ist H, OH, F oder Cl; und B und E sind unabhängig aus CH und N ausgewählt,

oder ein optisches oder geometrisches Isomer davon; oder ein pharmazeutisch annehmbares Salz, N-Oxid, Ester, quaternäres Ammoniumsalz oder Prodrug davon.

**3.** Verwendung nach Anspruch 1 oder 2, wobei der Östrogenagonist / -antagonist (-)-cis-6-Phenyl-5-[4-(2-pyrrolidin-1-yl-ethoxy)-phenyl]-5,6,7,8-tetrahydronaphthalin-2-ol oder ein optisches oder geometrisches Isomer davon oder ein pharmazeutisch annehmbares(r) Salz, N-Oxid, Ester, quaternäres Ammoniumsalz oder Prodrug davon ist.

**4.** Verwendung nach Anspruch 3, wobei der Östrogenagonist / antagonist in der Form eine D-Tartratsalzes vorliegt.

**5.** Verwendung einer therapeutisch wirksamen Menge einer Östrogenagonist- / -antagonistverbindung der Formel I

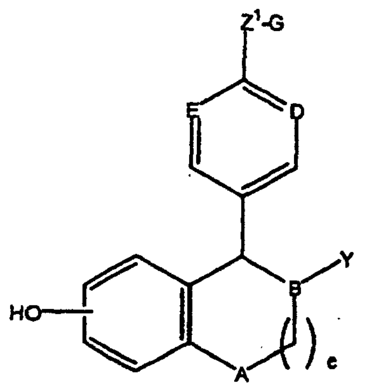

(I)

wobei gilt:

A ist aus $CH_2$ und NR ausgewählt;
B, D und E sind unabhängig aus CH und N ausgewählt;
Y ist

(a) ein optional mit 1-3 unabhängig aus $R^4$ ausgewählten Substituenten substituiertes Phenyl;
(b) ein optional mit 1-3 unabhängig aus $R^4$ ausgewählten Substituenten substituiertes Naphthyl;
(c) ein optional mit 1-2 unabhängig aus $R^4$ ausgewählten Substituenten substituiertes $C_3$-$C_8$-Cycloalkyl;
(d) ein optional mit 1-2 unabhängig aus $R^4$ ausgewählten Substituenten substituiertes $C_3$-$C_8$-Cycloalkenyl;
(e) ein fünfgliedriger, optional mit 1-3 unabhängig aus $R^4$ ausgewählten Substituenten substituierter Heterozyklus, welcher bis zu zwei aus der aus -O-, -$NR^2$- und -$S(O)_n$- bestehenden Gruppe ausgewählte Heteroatome aufweist;
(f) ein sechsgliedriger, optional mit 1-3 unabhängig aus $R^4$ ausgewählten Substituenten substituierter Heterozyklus, welcher bis zu zwei aus der aus -O-, -$NR^2$- und -$S(O)_n$- bestehenden Gruppe ausgewählte Heteroatome aufweist; oder

(g) ein aus einem fünf- oder sechsgliedrigen, mit einem Phenylring kondensierten heterozyklischen Ring bestehendes bizyklisches Ringsystem, wobei besagter heterozyklischer Ring bis zu zwei aus der aus -O-, $-NR^2-$ und $-S(O)_n-$ bestehenden Gruppe ausgewählte Heteroatome aufweist, das optional mit 1-3 unabhängig aus $R^4$ ausgewählten Substituenten substituiert ist;

$Z^1$ ist

(a) $-(CH_2)_pW(CH_2)_q-$;
(b) $-O(CH_2)_pCR^5R^6-$;
(c) $-O(CH_2)_pW(CH_2)_q-$;
(d) $-OCHR^2CHR^3-$; oder
(e) $-SCHR^2CHR^3-$;

G ist

(a) $-NR^7R^8$;
(b)

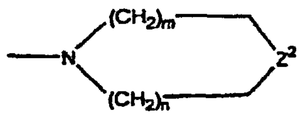

wobei n 0, 1 oder 2 ist; m 1, 2 oder 3 ist; $Z^2$ -NH-, -O-, -S- oder $-CH_2-$ ist; optional an benachbarten Kohlenstoffatomen mit einem oder zwei Phenylringen kondensiert und optional am Kohlenstoff unabhängig substituiert mit einem bis drei Substituenten und optional am Stickstoff unabhängig substituiert mit einem chemisch geeigneten Substituenten, ausgewählt aus $R^4$; oder (c) ein fünf bis zwölf Kohlenstoffatome enthaltendes bizyklisches Amin, das entweder verbrückt oder kondensiert ist und optional mit 1-3 unabhängig aus $R^4$ ausgewählten Substituenten substituiert ist; oder

$Z^1$ und G in Kombination können sein

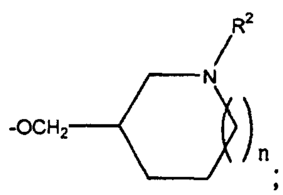

W ist

(a) $-CH_2-$;
(b) -CH=CH-;
(c) -O-;
(d) $-NR^2-$;
(e) $-S(O)_n-$;
(f)

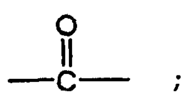

(g) $-CR^2(OH)-$;
(h) $-CONR^2$;
(i) $-NR^2CO-$;

(j)

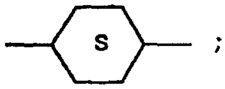

oder
(k) **-C≡C-** ;

R ist Wasserstoff oder $C_1$-$C_6$-Alkyl;
$R^2$ und $R^3$ sind unabhängig

(a) Wasserstoff; oder
(b) $C_1$-$C_4$-Alkyl;

$R^4$ ist

(a) Wasserstoff;
(b) Halogen;
(c) $C_1$-$C_6$-Alkyl;
(d) $C_1$-$C_4$-Alkoxy;
(e) $C_1$-$C_4$-Acyloxy;
(f) $C_1$-$C_4$-Alkylthio;
(g) $C_1$-$C_4$-Alkylsulfinyl;
(h) $C_1$-$C_4$-Alkylsulfonyl ;
(i) Hydroxy($C_1$-$C_4$)Alkyl;
(j) Aryl($C_1$-$C_4$)Alkyl;
(k) $-CO_2H$;
(l) -CN;
(m) -CONHOR;
(n) $-SO_2NHR$;
(o) $-NH_2$ ;
(p) $C_1$-$C_4$-Alkylamino ;
(q) $C_1$-$C_4$-Dialkylamino ;
(r) $-NHSO_2R$;
(s) $-NO_2$;
(t) -Aryl; oder
(u) -OH;

$R^5$ und $R^6$ sind unabhängig $C_1$-$C_8$-Alkyl oder bilden zusammen einen $C_3$-$C_{10}$-carbozyklischen Ring;
$R^7$ und $R^8$ sind unabhängig

(a) Phenyl;
(b) ein gesättigter oder ungesättigter $C_3$-$C_{10}$carbozyklischer Ring;
(c) ein bis zu zwei aus -O-, -N- und -S- ausgewählte Heteroatome enthaltender $C_3$-$C_{10}$-heterozyklischer Ring;
(d) H;
(e) $C_1$-$C_6$-Alkyl; oder
(f) bilden mit $R^5$ oder $R^6$ einen 3- bis 8-gliedrigen Stickstoff enthaltenden Ring;

$R^7$ und $R^8$ entweder als lineare Form oder Ringform können optional mit bis zu drei unabhängig aus $C_1$-$C_6$-Alkyl, Halogen, Alkoxy, Hydroxy und Carboxy ausgewählten Substituenten substituiert sein;
ein durch $R^7$ und $R^8$ gebildeter Ring ist optional mit einem Phenylring kondensiert;
e ist 0, 1 oder 2;
m ist 1,2 oder 3 ;
n ist 0, 1 oder 2;

p ist 0, 1, 2, oder 3;
q ist 0, 1, 2 oder 3;

oder eines optischen oder geometrischen Isomers davon; oder eines pharmazeutisch annehmbaren Salzes, N-Oxids, Esters, quaternären Ammoniumsalzes oder Prodrugs davon, und Testosteron zur Herstellung eines Medikamentes zur Behandlung von Gynäkomastie, Lipidstörungen, kardiovaskulären Erkrankungen, Atherosklerose, Hypogonadismus, benigner Prostatahyperplasie, Osteoprose, verminderter Libido oder verminderter vaskulärer Reaktivität in einem männlichen Patienten.

**6.** Verwendung nach Anspruch 5, wobei der Östrogenagonist / antagonist ist:

eine Verhindung der Formel (IA).

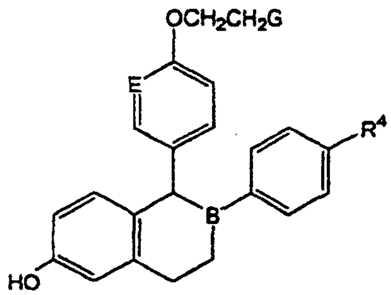

(IA)

wobei gilt
G ist

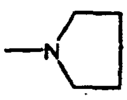 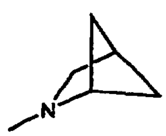 oder 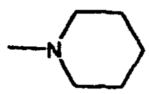 ;

$R^4$ ist H, OH, F oder Cl; und B und E sind unabhängig aus CH und N ausgewählt,

oder ein optisches oder geometrisches Isomer davon; oder ein pharmazeutisch annehmbares Salz, N-Oxid, Ester, quaternäres Ammoniumsalz oder Prodrug davon.

**7.** Verwendung nach Anspruch 5 oder 6, wobei der Östrogenagonist / -antagonist (-)-cis-6-Phenyl-5-[4-(2-pyrrolidin-1-yl-ethoxy)-phenyl]-5,6,7,8-tetrahydronaphthalin-2-ol oder ein optisches oder geometrisches Isomer davon, oder ein pharmazeutisch annehmbares(r) Salz, N-Oxid, Ester, quaternäres Ammoniumsalz oder Prodrug davon ist.

**8.** Verwendung nach Anspruch 7, wobei der Östrogenagonist / -antagonist in der Form eines D-Tartratsalzes vorliegt.

**9.** Kit zur Behandlung einer Abnahme des Spiegels des Hormons Testosteron bei einem männlichen Patienten, von Gynäkomastie, von Lipidstörungen, von kardiovaskulären Erkrankungen, von Atherosklerose, von Hypogonadismus, von benigner Prostatahyperplasie, von Osteoporose, von verminderter Libido oder verminderter vaskulärer Reaktivität in einem männlichen Patienten, welches Kit umfasst:

eine oder mehrere pharmazeutische Zusammensetzungen, enthaltend einen Östrogenagonisten / -antagonisten und Testosteron; und

wobei der Östrogenagonist / -antagonist eine Verbindung der Formel I ist,

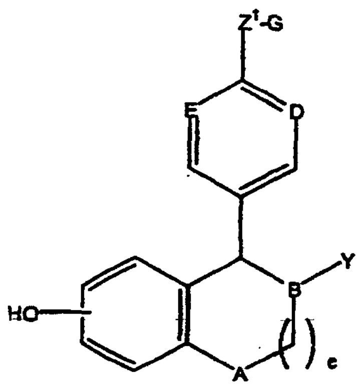

(I)

wobei gilt:

A ist aus $CH_2$ und NR ausgewählt;
B, D und E sind unabhängig aus CH und N ausgewählt;
Y ist

(a) ein optional mit 1-3 unabhängig aus $R^4$ ausgewählten Substituenten substituiertes Phenyl;
(b) ein optional mit 1-3 unabhängig aus $R^4$ ausgewählten Substituenten substituiertes Naphthyl;
(c) ein optional mit 1-2 unabhängig aus $R^4$ ausgewählten Substituenten substituiertes $C_3$-$C_8$-Cycloalkyl;
(d) ein optional mit 1-2 unabhängig aus $R^4$ ausgewählten Substituenten substituiertes $C_3$-$C_8$-Cycloalkenyl;
(e) ein fünfgliedriger, optional mit 1-3 unabhängig aus $R^4$ ausgewählten Substituenten substituierter Heterozyklus, welcher bis zu zwei aus der aus -O-, -$NR^2$- und -$S(O)_n$- bestehenden Gruppe ausgewählte Heteroatome aufweist;
(f) ein sechsgliedriger, optional mit 1-3 unabhängig aus $R^4$ ausgewählten Substituenten substituierter Heterozyklus, welcher bis zu zwei aus der aus -O-, -$NR^2$- und -$S(O)_n$- bestehenden Gruppe ausgewählte Heteroatome aufweist; oder
(g) ein aus einem fünf- oder sechsgliedrigen, mit einem Phenylring kondensierten heterozyklischen Ring bestehendes bizyklisches Ringsystem, wobei besagter heterozyklischer Ring bis zu zwei aus der aus -O-, -$NR^2$- und -$S(O)_n$- bestehenden Gruppe ausgewählte Heteroatome aufweist, das optional mit 1-3 unabhängig aus $R^4$ ausgewählten Substituenten substituiert ist;

$Z^1$ ist

(a) $-(CH_2)_pW(CH_2)_q-$;
(b) $-O(CH_2)_pCR^5R^6-$;
(c) $-O(CH_2)_pW(CH_2)_q-$;
(d) $-OCHR^2CHR^3-$; oder
(e) $-SCHR^2CHR^3-$;

G ist

(a) $-NR^7R^8$;
(b)

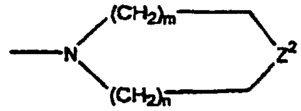

wobei n 0, 1 oder 2 ist; m 1, 2 oder 3 ist; $Z^2$ -NH-, -O-, -S- oder $-CH_2-$ ist; optional an benachbarten Kohlenstoffatomen mit einem oder zwei Phenylringen kondensiert und optional am Kohlenstoff unabhängig substituiert mit einem bis drei Substituenten und optional am Stickstoff unabhängig substituiert mit einem chemisch geeigneten Substituenten, ausgewählt aus $R^4$; oder

(c) ein fünf bis zwölf Kohlenstoffatome enthaltendes bizyklisches Amin, das entweder verbrückt oder kondensiert ist und optional mit 1-3 unabhängig aus $R^4$ ausgewählten Substituenten substituiert ist; oder

$Z^1$ und G in Kombination können sein

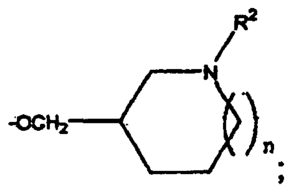

W ist

(a) $-CH_2-$;
(b) -CH=CH-;
(c) -O-;
(d) $-NR^2-$;
(e) $-S(O)_n-$;
(f)

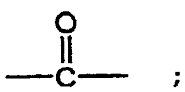

(g) $-CR^2$ (OH) -;
(h) - $CONR^2$;
(i) $-NR^2CO-$;
(j)

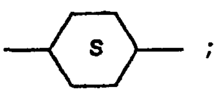

oder
(k) -C≡C-;

R ist Wasserstoff oder $C_1$-$C_6$-Alkyl;
$R^2$ und $R^3$ sind unabhängig

(a) Wasserstoff; oder
(b) $C_1$-$C_4$-Alkyl;

$R^4$ ist

(a) Wasserstoff;
(b) Halogen;
(c) $C_1$-$C_6$-Alkyl;
(d) $C_1$-$C_4$-Alkoxy;
(e) $C_1$-$C_4$-Acyloxy;
(f) $C_1$-$C_4$-Alkylthio;

(g) $C_1$-$C_4$-Alkylsulfinyl;
(h) $C_1$-$C_4$-Alkylsulfonyl;
(i) Hydroxy($C_1$-$C_4$)Alkyl;
(j) Aryl($C_1$-$C_4$)Alkyl;
(k) -$CO_2H$;
(l) -CN;
(m) -CONHOR;
(n) -$SO_2NHR$;
(o) -$NH_2$;
(p) $C_1$-$C_4$-Alkylamino;
(q) $C_1$-$C_4$-Dialkylamino;
(r) -$NHSO_2R$;
(s) -$NO_2$;
(t) -Aryl; oder
(u) -OH;

$R^5$ und $R^6$ sind unabhängig $C_1$-$C_8$-Alkyl oder bilden zusammen einen $C_3$-$C_{10}$-carbozyklischen Ring;
$R^7$ und $R^8$ sind unabhängig

(a) Phenyl;
(b) ein gesättigter oder ungesättigter $C_3$-$C_{10}$carbozyklischer Ring;
(c) ein bis zu zwei aus -O-, -N- und -S- ausgewählte Heteroatome enthaltender $C_3$-$C_{10}$-heterozyklischer Ring;
(d) H;
(e) $C_1$-$C_6$-Alkyl; oder
(f) bilden mit $R^5$ oder $R^6$ einen 3- bis 8-gliedrigen, Stickstoff enthaltenden Ring;

$R^7$ und $R^8$ entweder als lineare Form oder Ringform können optional mit bis zu drei unabhängig aus $C_1$-$C_6$-Alkyl, Halogen, Alkoxy, Hydroxy und Carboxy ausgewählten Substituenten substituiert sein;
ein durch $R^7$ und $R^8$ gebildeter Ring ist optional mit einem Phenylring kondensiert;
e ist 0, 1 oder 2;
m ist 1,2 oder 3;
n ist 0, 1 oder 2;
p ist 0, 1, 2, oder 3;
q ist 0, 1, 2 oder 3;

oder ein optisches oder geometrisches Isomer davon; oder ein pharmazeutisch annehmbares(r) Salz, N-Oxid, Ester, quaternäres Ammoniumsalz oder Prodrug davon.

**10.** Kit nach Anspruch 9, wobei der Östrogenagonist / -antagonist ist:

eine Verbindung der Formel (IA),
wobei gilt

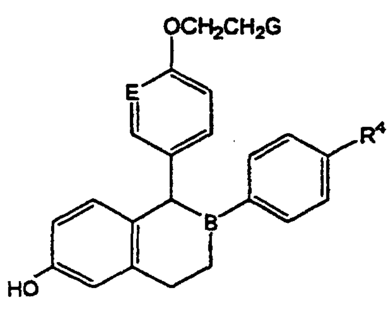

(IA)

wobei gilt:

G ist

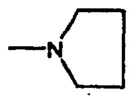 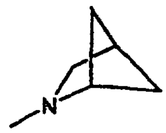 oder 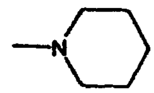 ;

$R^4$ ist H, OH, F oder Cl; und B und E sind unabhängig aus CH und N ausgewählt,

oder ein optisches oder geometrisches Isomer davon; oder ein pharmazeutisch annehmbares(r) Salz, N-Oxid, Ester, quaternäres Ammoniumsalz oder Prodrug davon.

**11.** Kit nach Anspruch 9 oder 10, wobei der Östrogenagonist / -antagonist (-)-cis-6-Phenyl-5-[4-(2-pyrrolidin-1-ylethoxy)-phenyl]-5,6,7,8-tetrahydronaphthalin-2-ol oder ein optisches oder geometrisches Isomer davon oder ein pharmazeutisch annehmbares(r) Salz, N-Oxid, Ester, quaternäres Ammoniumsalz oder Prodrug davon ist.

**12.** Kit nach Anspruch 11, wobei der Östrogenagonist / -antagonist in der Form eines D-Tartratsalzes vorliegt.

**13.** Kombination einer Verbindung der Formel I wie in Anspruch 1 definiert, oder eines optischen oder geometrischen Isomeren davon; oder eines pharmazeutisch annehmbaren Salzes, N-Oxids, Esters, quaternären Ammoniumsalzes oder Prodrugs davon und Testosteron zur Verwendung als ein Mittel zur Behandlung des Testosteronmangels bei einem männlichen Patienten.

**14.** Pharmazeutische Produkte, enthaltend eine Verbindung der Formel I wie in Anspruch 1 definiert, oder ein optisches oder geometrisches Isomer davon; oder ein pharmazeutisch annehmbares(r) Salz, N-Oxid, Ester, quaternäres Ammoniumsalz oder Prodrug davon und Testosteron als eine kombinierte Zubereitung zur simultanen, separaten oder sequenziellen Verwendung zur Behandlung des Testosteronmangels bei einem männlichen Patienten.

**Revendications**

**1.** Utilisation d'une quantité thérapeutiquement efficace d'un composé agoniste/antagoniste des oestrogènes de formule I :

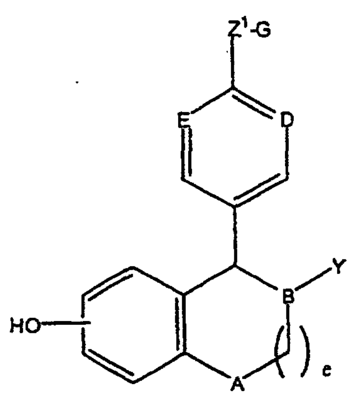

(I)

dans laquelle :

A est sélectionné entre $CH_2$ et NR ;

B, D et E sont indépendamment sélectionnés entre CH et N ;
Y représente :

(a) phényle, optionnellement substitué par 1-3 substituants indépendamment sélectionnés parmi $R^4$ ;
(b) naphtyle, optionnellement substitué par 1-3 substituants indépendamment sélectionnés parmi $R^4$ ;
(c) cycloalkyle en $C_3$-$C_8$, optionnellement substitué par 1-2 substituants indépendamment sélectionnés parmi $R^4$ ;
(d) cycloalkényle en $C_3$-$C_8$, optionnellement substitué par 1-2 substituants indépendamment sélectionnés parmi $R^4$ ;
(e) un hétérocycle à cinq éléments contenant jusqu'à deux hétéroatomes sélectionnés dans le groupe consistant en -O-, $-NR^2-$ et $-S(O)_n-$ optionnellement substitué par 1-3 substituants indépendamment sélectionnés parmi $R^4$ ; ou
(f) un hétérocycle à six éléments contenant jusqu'à deux hétéroatomes sélectionnés dans le groupe consistant en -O-, $-NR^2-$ et $-S(O)_n-$ optionnellement substitué par 1-3 substituants indépendamment sélectionnés parmi $R^4$ ; ou
(g) un système de noyau bicyclique consistant en un noyau hétérocyclique à cinq ou six éléments fusionné à un noyau phényle, ledit noyau hétérocyclique contenant jusqu'à deux hétéroatomes sélectionnés dans le groupe consistant en -O-, $-NR^2-$ et $-S(O)_n-$, optionnellement substitué par 1-3 substituants indépendamment sélectionnés parmi $R^4$ ;

$Z^1$ représente :

(a) $-(CH_2)_p W(CH_2)_q-$ ;
(b) $-O(CH_2)_p CR^5R^6-$ ;
(c) $-O(CH_2)_p W(CH_2)_q-$
(d) $-OCHR^2CHR^3-$ ; ou
(e) $-SCHR^2CHR^3-$ ;

G représente :

(a) $-NR^7R^8$ ;
(b)

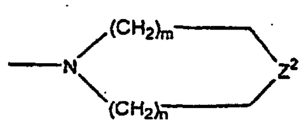

dans laquelle n représente 0, 1 ou 2 ; m représente 1, 2 ou 3 ; $Z_2$ représente -NH-, -O-, -S- ou $-CH_2-$ ; optionnellement fusionné sur des atomes de carbone adjacents avec un ou deux noyaux phényle et, optionnellement, indépendamment substitué sur le carbone par de un à trois substituants et, optionnellement, indépendamment substitué sur l'azote par un substituant, chimiquement convenable, sélectionné parmi $R^4$ ; ou
(c) une amine bicyclique contenant de cinq à douze atomes de carbone, soit pontée, soit fusionnée, et optionnellement substituée par 1-3 substituants indépendamment sélectionnés parmi $R^4$ ; ou

$Z^1$ et G, en combinaison, peuvent représenter

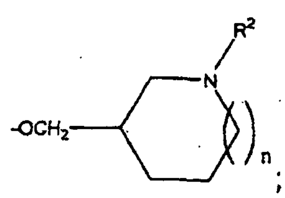
;

W représente :

(a) $-CH_2-$ ;
(b) -CH=CH- ;
(c) -O- ;
(d) $-NR^2-$ ;
(e) $-S(O)_n-$ ;
(f)

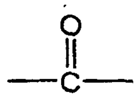

(g) $-CR^2$ (OH) - ;
(h) $-CONR^2-$ ;
(i) $-NR^2CO-$ ;
(j)

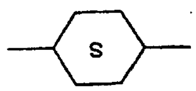

ou
(k) -C≡C- ;

R représente l'hydrogène ou alkyle en $C_1$-$C_6$;
$R^2$ et $R^3$ représentent, indépendamment :

(a) l'hydrogène ; ou
(b) alkyle en $C_1$-$C_4$ ;

$R^4$ représente :

(a) l'hydrogène ;
(b) halogèno ;
(c) alkyle en $C_1$-$C_6$ ;
(d) (alkyle en $C_1$-$C_4$)oxy ;
(e) (acyle en $C_1$-$C_4$)oxy ;
(f) (alkyle en $C_1$-$C_4$) thio ;
(g) (alkyle en $C_1$-$C_4$)sulfinyle ;
(h) (alkyle en $C_1$-$C_4$)sulfonyle ;
(i) hydroxy(alkyle en $C_1$-$C_4$ ) ;
(j) aryl(alkyle en $C_1$-$C_4$ ) ;
(k) $-CO_2H$ ;
(l) -CN ;
(m) -CONHOR ;
(n) $-SO_2NHR$ ;
(o) $-NH_2$ ;
(p) (alkyle en $C_1$-$C_4$) amino ;
(q) (dialkyle en $C_1$-$C_4$)amino ;
(r) $-NHSO_2R$ ;
(s) $-NO_2$ ;
(t) -aryle ; ou
(u) -OH ;

$R^5$ et $R^6$ représentent, indépendamment, alkyle en $C_1$-$C_8$ ou, ensemble, forment un noyau carbocyclique en

$C_3$-$C_{10}$ ;
$R^7$ et $R^8$ représentent, indépendamment :

(a) phényle ;
(b) un noyau carbocyclique en $C_3$-$C_{10}$, saturé ou insaturé ;
(c) un noyau hétérocyclique en $C_3$-$C_{10}$ contenant jusqu'à deux hétéroatomes sélectionnés parmi -O-, -N- et -S- ;
(d) H ;
(e) (alkyle en $C_1$-$C_6$) ; ou
(f) forment, avec $R^5$ ou $R^6$, un noyau ayant de 3 à 8 éléments et contenant de l'azote ;

$R^7$ et $R^8$, soit sous la forme linéaire, soit sous la forme cyclique, peuvent optionnellement être substitués par jusqu'à trois substituants indépendamment sélectionnés parmi alkyle en $C_1$-$C_6$, halogène, alkoxy, hydroxy et carboxy ;
un noyau formé par $R^7$ et $R^8$ peut être optionnellement fusionné à un noyau phényle ;
e représente 0, 1 ou 2 ;
m représente 1, 2 ou 3 ;
n représente 0, 1 ou 2 ;
p représente 0, 1, 2 ou 3 ;
q représente 0, 1, 2 ou 3 ;

ou un isomère optique ou géométrique d'un tel composé; ou un sel, un N-oxyde, un ester, un sel d'ammonium quaternaire pharmaceutiquement acceptables d'un tel composé, ou un pro-médicament d'un tel composé, et de la testostérone pour la fabrication d'un médicament pour le traitement d'un déclin du niveau de l'hormone testostérone chez un patient mâle.

**2.** Utilisation selon la revendication 1, dans laquelle l'agoniste/antagoniste des oestrogènes est un composé de formule (IA) :

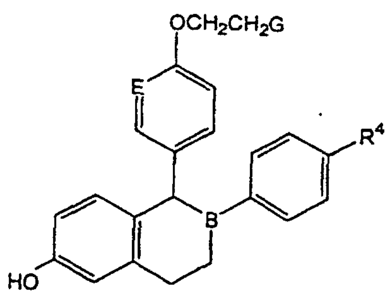

(IA)

dans laquelle G représente

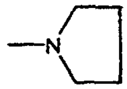

, 

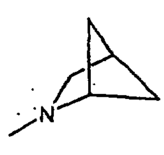

ou

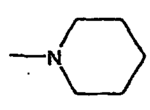

$R^4$ représente H, OH, F ou Cl; et B et E sont indépendamment sélectionnés parmi CH et N, ou un isomère optique ou géométrique d'un tel composé ; ou un sel, un N-oxyde, un ester, un sel d'ammonium quaternaire pharmaceutiquement acceptables d'un tel composé, ou un pro-médicament d'un tel composé.

**3.** Utilisation selon la revendication 1 ou 2, dans laquelle l'agoniste/antagoniste des oestrogènes est le (-)-cis-6-phényl-5-[4-(2-pyrrolidin-1-yl-éthoxy)-phényl]-5,6,7,8-tétrahydronaphtalène-2-ol ou un isomère optique ou géométrique d'un tel composé ; ou un sel, un N-oxyde, un ester, un sel d'ammonium quaternaire pharmaceutiquement acceptables d'un tel composé, ou un pro-médicament d'un tel composé.

4. Utilisation selon la revendication 3, dans laquelle l'agoniste/antagoniste des oestrogènes est sous la forme d'un sel D-tartrate.

5. Utilisation d'une quantité thérapeutiquement efficace d'un composé agoniste/antagoniste des oestrogènes. selon la formule I

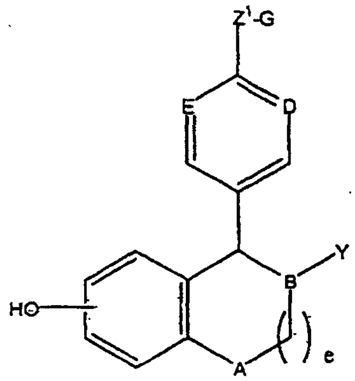

(I)

dans laquelle

A est sélectionné entre $CH_2$ et NR ;
B, D et E sont indépendamment sélectionnés entre CH et N ;
Y représente :

(a) phényle, optionnellement substitué par 1-3 substituants indépendamment sélectionnés parmi $R^4$ ;
(b) naphtyle, optionnellement substitué par 1-3 substituants indépendamment sélectionnés parmi $R^4$ ;
(c) cycloalkyle en $C_3$-$C_8$, optionnellement substitué par 1-2 substituants indépendamment sélectionnés parmi $R^4$ ;
(d) cycloalkényle en $C_3$-$C_8$, optionnellement substitué par 1-2 substituants indépendamment sélectionnés parmi $R^4$ ;
(e) un hétérocycle à cinq éléments contenant jusqu'à deux hétéroatomes sélectionnés dans le groupe consistant en -O-, $-NR^2-$ et $-S(O)_n-$ optionnellement substitué par 1-3 substituants indépendamment sélectionnés parmi $R^4$ ; ou
(f) un hétérocycle à six éléments contenant jusqu'à deux hétéroatomes sélectionnés dans le groupe consistant en -O-, $-NR^2-$ et $-S(O)_n-$ optionnellement substitué par 1-3 substituants indépendamment sélectionnés parmi $R^4$ ; ou
(g) un système de noyau bicyclique consistant en un noyau hétérocyclique à cinq ou six éléments fusionné à un noyau phényle, ledit noyau hétérocyclique contenant jusqu'à deux hétéroatomes sélectionnés dans le groupe consistant en -O-, $-NR^2-$ et $-S(O)_n-$, optionnellement substitué par 1-3 substituants indépendamment sélectionnés parmi $R^4$ ;

$Z^1$ représente :

(a) $-(CH_2)_p W(CH_2)_q-$ ;
(b) $-O(CH_2)_p CR^5R^6-$;
(c) $-O(CH_2)_p W(CH_2)_q-$
(d) $-OCHR^2CHR^3-$ ; ou
(e) $-SCHR^2CHR^3-$ ;

G représente :

(a) $-NR^7R^8$ ;

(b)

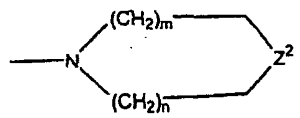

dans laquelle n représente 0, 1 ou 2 ; m représente 1, 2 ou 3 ; $Z_2$ représente -NH-, -O-, -S- ou $-CH_2-$ ; optionnellement fusionné sur des atomes de carbone adjacents avec un ou deux noyaux phényle et, optionnellement, indépendamment substitué sur le carbone par de un à trois substituants et, optionnellement, indépendamment substitué sur l'azote par un substituant, chimiquement convenable, sélectionné parmi $R^4$ ; ou
(c) une amine bicyclique contenant de cinq à douze atomes de carbone, soit pontée, soit fusionnée, et optionnellement substituée par 1-3 substituants indépendamment sélectionnés parmi $R^4$ ; ou

$Z^1$ et G, en combinaison, peuvent représenter

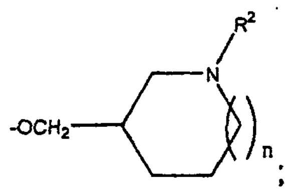

W représente :

(a) $-CH_2-$ ;
(b) -CH=CH- ;
(c) -O- ;
(d) $-NR^2-$ ;
(e) $-S(O)_n-$ ;
(f)

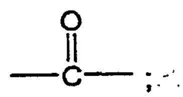

(g) $-CR^2(OH)-$ ;
(h) $-CONR^2-$ ;
(i) $-NR^2CO-$ ;
(j)

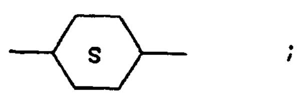

ou
(k) -C≡C- ;

R représente l'hydrogène ou alkyle en $C_1$-$C_6$ ;
$R^2$ et $R^3$ représentent, indépendamment :

(a) l'hydrogène ; ou
(b) alkyle en $C_1$-$C_4$ ;

$R^4$ représente :

(a) l'hydrogène ;
(b) halogène ;
(c) alkyle en $C_1$-$C_6$ ;
(d) (alkyle en $C_1$-$C_4$)oxy;
(e) (acyle en $C_1$-$C_4$)oxy ;
(f) (alkyle en $C_1$-$C_4$)thio;
(g) (alkyle en $C_1$-$C_4$)sulfinyle ;
(h) (alkyle en $C_1$-$C_4$)sulfonyle ;
(i) hydroxy(alkyle en $C_1$-$C_4$) ;
(j) aryl (alkyle en $C_1$-$C_4$ ) ;
(k) -$CO_2H$ ;
(l) -CN ;
(m) -CONHOR ;
(n) -$SO_2NHR$ ;
(o) -$NH_2$ ;
(p) (alkyle en $C_1$-$C_4$)amino ;
(q) (dialkyle en $C_1$-$C_4$)amino ;
(r) -$NHSO_2R$ ;
(s) -$NO_2$ ;
(t) -aryle ; ou
(u) -OH ;

$R^5$ et $R^6$ représentent, indépendamment, alkyle en $C_1$-$C_8$ ou, ensemble, forment un noyau carbocyclique en $C_3$-$C_{10}$ ;

$R^7$ et $R^8$ représentent, indépendamment :

(a) phényle ;
(b) un noyau carbocyclique en $C_3$-$C_{10}$, saturé ou insaturé ;
(c) un noyau hétérocyclique en $C_3$-$C_{10}$ contenant jusqu'à deux hétéroatomes sélectionnés parmi -O-, -N- et -S- ;
(d) H ;
(e) (alkyle en $C_1$-$C_6$) ; ou
(f) forment, avec $R^5$ ou $R^6$, un noyau ayant de 3 à 8 éléments et contenant de l'azote ;

$R^7$ et $R^8$, soit sous la forme linéaire, soit sous la forme cyclique, peuvent optionnellement être substitués par jusqu'à trois substituants indépendamment sélectionnés parmi alkyle en $C_1$-$C_6$, halogéno, alkoxy, hydroxy et carboxy ;

un noyau formé par $R^7$ et $R^8$ peut être optionnellement fusionné à un noyau phényle ;
e représente 0, 1 ou 2 ;
m représente 1, 2 ou 3 ;
n représente 0, 1 ou 2 ;
p représente 0, 1, 2 ou 3 ;
q représente 0, 1, 2 ou 3 ;

ou un isomère optique ou géométrique d'un tel composé; ou un sel, un N-oxyde, un ester, un sel d'ammonium quaternaire pharmaceutiquement acceptables d'un tel composé, ou un pro-médicament d'un tel composé, pour la fabrication d'un médicament pour le traitement de la gynécomastie, des troubles lipidiques, de la maladie cardio-vasculaire, de l'athérosclérose, de l'hypogonadisme, de l'hyperplasie bénigne de la prostate, de l'ostéoporose, de la libido réduite, ou de la réactivité vasculaire réduite chez un patient mâle.

**6.** Utilisation selon la revendication 5,dans laquelle l'agoniste/antagoniste des oestrogènes est un composé de formule (IA) :

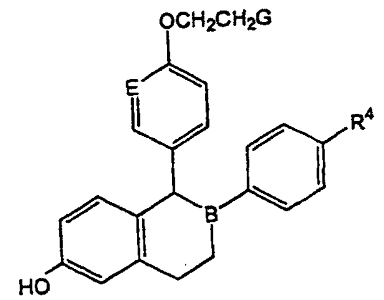

(IA)

dans laquelle G représente

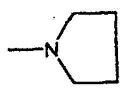, 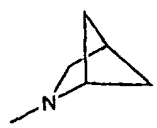 ou 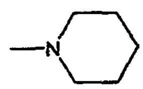 ;

$R^4$ représente H, OH, F ou Cl ; et B et E sont indépendamment sélectionnés parmi CH et N, ou un isomère optique ou géométrique d'un tel composé ; ou un sel, un N-oxyde, un ester, un sel d'ammonium quaternaire pharmaceutiquement acceptables d'un tel composé, ou un pro-médicament d'un tel composé.

7. Utilisation selon la revendication 5 ou 6, dans laquelle dans laquelle l'agoniste/antagoniste des oestrogènes est le (-)-cis-6-phényl-5-[4-(2-pyrrolidin-1-yl-éthoxy)-phényl]-5,6,7,8-tétrahydronaphtalène-2-ol ou un isomère optique ou géométrique d'un tel composé ; ou un sel, un N-oxyde, un ester, un sel d'ammonium quaternaire pharmaceutiquement acceptables d'un tel composé, ou un pro-médicament d'un tel composé.

8. Utilisation selon la revendication 7, dans laquelle l'agoniste/antagoniste des oestrogènes est sous la forme d'un sel D-tartrate.

9. Kit pour le traitement d'un déclin du niveau de l'hormone testostérone chez un patient mâle, de la gynécomastie, des troubles lipidiques, de la maladie cardio-vasculaire, de l'athérosclérose, de l'hypogonadisme, de l'hyperplasie bénigne de la prostate, de l'ostéoporose, de la libido réduite, ou de la réactivité vasculaire réduite chez un patient mâle, le kit comprenant :

une ou plusieurs compositions pharmaceutiques comprenant un agoniste/antagoniste des oestrogènes et de la testostérone ; et
dans laquelle l'agoniste/antagoniste des oestrogènes est un composé de formule I

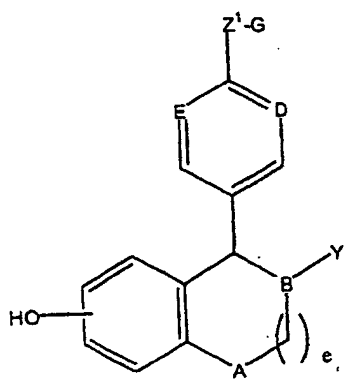

(I)

dans laquelle

A est sélectionné entre $CH_2$ et NR ; B, D et E sont indépendamment sélectionnés entre CH et N ;

Y représente :

(a) phényle, optionnellement substitué par 1-3 substituants indépendamment sélectionnés parmi $R^4$ ;
(b) naphtyle, optionnellement substitué par 1-3 substituants indépendamment sélectionnés parmi $R^4$ ;
(c) cycloalkyle en $C_3$-$C_8$, optionnellement substitué par 1-2 substituants indépendamment sélectionnés parmi $R^4$;
(d) cycloalkényle en $C_3$-$C_8$, optionnellement substitué par 1-2 substituants indépendamment sélectionnés parmi $R^4$ ;
(e) un hétérocycle à cinq éléments contenant jusqu'à deux hétéroatomes sélectionnés dans le groupe consistant en -O-, -$NR^2$- et -$S(O)_n$- optionnellement substitué par 1-3 substituants indépendamment sélectionnés parmi $R^4$ ; ou
(f) un hétérocycle à six éléments contenant jusqu'à deux hétéroatomes sélectionnés dans le groupe consistant en -O-, -$NR^2$- et -$S(O)_n$- optionnellement substitué par 1-3 substituants indépendamment sélectionnés parmi $R^4$ ; ou
(g) un système de noyau bicyclique consistant en un noyau hétérocyclique à cinq ou six éléments fusionné à un noyau phényle, ledit noyau hétérocyclique contenant jusqu'à deux hétéroatomes sélectionnés dans le groupe consistant en -O-, -$NR^2$- et -$S(O)_n$-, optionnellement substitué par 1-3 substituants indépendamment sélectionnés parmi $R^4$ ;

$Z^1$ représente :

(a) -$(CH_2)_p$ $W(CH_2)_q$- ;
(b) -$O(CH_2)_p$ $CR^5R^6$- ;
(c) -$O(CH_2)_p$ $W(CH_2)_q$-
(d) -$OCHR^2CHR^3$-; ou
(e) -$SCHR^2CHR^3$- ;

G représente :

(a) -$NR^7R^8$ ;
(b)

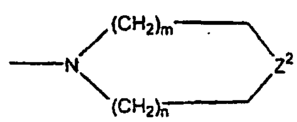

dans laquelle n représente 0, 1 ou 2 ; m représente 1, 2 ou 3 ; $Z_2$ représente -NH-, -O-, -S- ou -$CH_2$- ; optionnellement fusionné sur des atomes de carbone adjacents avec un ou deux noyaux phényle et, optionnellement, indépendamment substitué sur le carbone par de un à trois substituants et, optionnellement, indépendamment substitué sur l'azote par un substituant, chimiquement convenable, sélectionné parmi $R^4$ ; ou
(c) une amine bicyclique contenant de cinq à douze atomes de carbone, soit pontée, soit fusionnée, et optionnellement substituée par 1-3 substituants indépendamment sélectionnés parmi $R^4$ ; ou

$Z^1$ et G, en combinaison, peuvent représenter

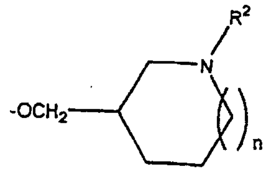

;

W représente :

(a) $-CH_2-$ ;
(b) -CH=CH- ;
(c) -O- ;
(d) $-NR^2-$ ;
(e) $-S(O)_n-$ ;
(f)

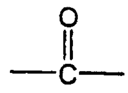 ;

(g) $-CR^2(OH)-$ ;
(h) $-CONR^2-$ ;
(i) $-NR^2CO-$ ;
(j)

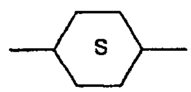 ;

ou
(k) $-C{\equiv}C-$ ;

R représente l'hydrogène ou alkyle en $C_1$-$C_6$ ;
$R^2$ et $R^3$ représentent, indépendamment :

(a) l'hydrogène ; ou
(b) alkyle en $C_1$-$C_4$ ;

$R^4$ représente :

(a) l'hydrogène ;
(b) halogèno ;
(c) alkyle en $C_1$-$C_6$ ;
(d) (alkyle en $C_1$-$C_4$)oxy;
(e) (acyle en $C_1$-$C_4$) oxy ;
(f) (alkyle en $C_1$-$C_4$) thio ;
(g) (alkyle en $C_1$-$C_4$) sulfinyle ;
(h) (alkyle en $C_1$-$C_4$)sulfonyle ;
(i) hydroxy(alkyle en $C_1$-$C_4$ ) ;
(j) aryl(alkyle en $C_1$-$C_4$) ;
(k) $-CO_2H$ ;
(l) -CN ;
(m) -CONHOR ;
(n) $-SO_2NHR$ ;
(o) $-NH_2$ ;
(p) (alkyle en $C_1$-$C_4$)amino ;
(q) (dialkyle en $C_1$-$C_4$)amino ;
(r) $-NHSO_2R$ ;
(s) $-NO_2$ ;
(t) -aryle ; ou
(u) -OH ;

$R^5$ et $R^6$ représentent, indépendamment, alkyle en $C_1$-$C_8$ ou, ensemble, forment un noyau carbocyclique

en $C_3$-$C_{10}$;

$R^7$ et $R^8$ représentent, indépendamment :

(a) phényle ;
(b) un noyau carbocyclique en $C_3$-$C_{10}$, saturé ou insaturé ;
(c) un noyau hétérocyclique en $C_3$-$C_{10}$ contenant jusqu'à deux hétéroatomes sélectionnés parmi -O-, -N- et -S- ;
(d) H ;
(e) (alkyle en $C_1$-$C_6$) ; ou
(f) forment, avec $R^5$ ou $R^6$, un noyau ayant de 3 à 8 éléments et contenant de l'azote ;

$R^7$ et $R^8$, soit sous la forme linéaire, soit sous la forme cyclique, peuvent optionnellement être substitués par jusqu'à trois substituants indépendamment sélectionnés parmi alkyle en $C_1$-$C_6$, halogèno, alkoxy, hydroxy et carboxy ;

un noyau formé par $R^7$ et $R^8$ peut être optionnellement fusionné à un noyau phényle ;
e représente 0, 1 ou 2 ;
m représente 1, 2 ou 3 ;
n représente 0, 1 ou 2 ;
p représente 0, 1, 2 ou 3 ;
q représente 0, 1, 2 ou 3 ;

ou un isomère optique ou géométrique d'un tel composé; ou un sel, un N-oxyde, un ester, un sel d'ammonium quaternaire pharmaceutiquement acceptables d'un tel composé, ou un pro-médicament d'un tel composé.

**10.** Kit selon la revendication 9, dans laquelle dans laquelle l'agoniste/antagoniste des oestrogènes est un composé de formule (IA) :

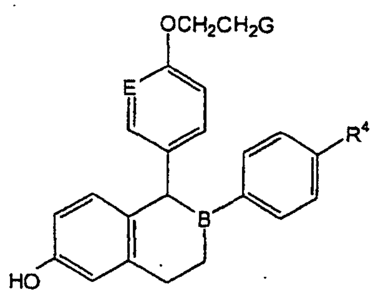

dans laquelle G représente

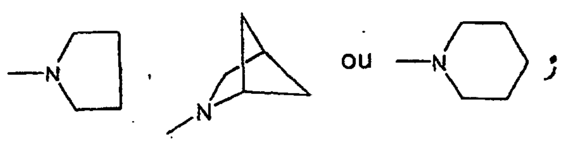

$R^4$ représente H, OH, F ou Cl ; et B et E sont indépendamment sélectionnés parmi CH et N, ou un isomère optique ou géométrique d'un tel composé ; ou un sel, un N-oxyde, un ester, un sel d'ammonium quaternaire pharmaceutiquement acceptables d'un tel composé, ou un pro-médicament d'un tel composé.

**11.** Kit selon la revendication 9 ou 10, dans laquelle dans laquelle l'agoniste/antagoniste des oestrogènes est le (-)-cis-6-phényl-5-[4-(2-pyrrolidin-1-yl-éthoxy)-phényl]-5,6,7,8-tétrahydronaphtalène-2-ol ou un isomère optique ou géométrique d'un tel composé ; ou un sel, un N-oxyde, un ester, un sel d'ammonium quaternaire pharmaceutiquement acceptables d'un tel composé, ou un pro-médicament d'un tel composé.

**12.** Kit selon la revendication 11, dans laquelle l'agoniste/antagoniste des oestrogènes est sous la forme d'un sel D-tartrate.

**13.** Combinaison d'un composé de formule I, selon la revendication 1, ou d'un isomère optique ou géométrique d'un tel composé; ou d'un sel, d'un N-oxyde, d'un ester, d'un sel d'ammonium quaternaire pharmaceutiquement acceptables d'un tel composé, ou d'un pro-médicament d'un tel composé, et de la testostérone pour une utilisation comme agent de traitement d'une déficience en testostérone chez un patient mâle.

**14.** Produits pharmaceutiques contenant un composé de formule I, tel que défini dans la revendication 1, ou un ou un isomère optique ou géométrique d'un tel composé; ou un sel, un N-oxyde, un ester, un sel d'ammonium quaternaire pharmaceutiquement acceptables d'un tel composé, ou un pro-médicament d'un tel composé, et de la testostérone sous la forme d'une préparation combinée pour l'utilisation simultanée, séparée ou séquentielle dans le traitement d'une déficience en testostérone chez un patient mâle.